(19) Europäisches Patentamt
European Patent Office
Office européen des brevets

(11) **EP 3 047 013 B1**

(12) **EUROPEAN PATENT SPECIFICATION**

(45) Date of publication and mention
of the grant of the patent:
**18.08.2021 Bulletin 2021/33**

(21) Application number: **14780959.4**

(22) Date of filing: **16.09.2014**

(51) Int Cl.:
**C12M 1/00** $^{(2006.01)}$

(86) International application number:
**PCT/US2014/055897**

(87) International publication number:
**WO 2015/039115 (19.03.2015 Gazette 2015/11)**

(54) **METHODS AND SYSTEMS FOR PROCESSING A CELL CULTURE**

VERFAHREN UND SYSTEME ZUR VERARBEITUNG EINER ZELLKULTUR

PROCÉDÉS ET SYSTÈMES DE TRAITEMENT D'UNE CULTURE CELLULAIRE

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**

(30) Priority: **16.09.2013 US 201361878502 P**

(43) Date of publication of application:
**27.07.2016 Bulletin 2016/30**

(73) Proprietor: **Genzyme Corporation
Cambridge, MA 02142 (US)**

(72) Inventors:
• **ZHOU, Hang**
  **Shanghai 201323 (CN)**
• **WRIGHT, Benjamin**
  **Bridgewater, New Jersey 08807 (US)**
• **YU, Marcella**
  **Bridgewater, Massachusetts 08807 (US)**
• **YIN, Jin**
  **Bridgewater, New Jersey 08807 (US)**
• **KONSTANTINOV, Konstantin**
  **Bridgewater, New Jersey 08807 (US)**

(74) Representative: **Lavoix
Bayerstraße 83
80335 München (DE)**

(56) References cited:
**WO-A1-2014/051503      US-A1- 2011 011 486
US-A1- 2011 111 486      US-B1- 6 544 424**

• **"Conversion of Bioreactors to Continuous
Perfusion Using Hollow Fiber Cell Separators", ,
1 January 1989 (1989-01-01), XP055155533,
Retrieved from the Internet:
URL:http://www.spectrumlabs.com/lit/CPAG.p
df [retrieved on 2014-11-27]**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

## Description

### TECHNICAL FIELD

This invention relates to methods of processing a cell culture and biotechnology, and more specifically, to methods of continuously processing a cell culture in a perfusion bioreactor.

### BACKGROUND

Mammalian cells are often used to produce therapeutic proteins. In some processing methods, mammalian cells are cultured in a perfusion bioreactor, a volume of cell culture containing the recombinant protein is removed from the bioreactor, and new culture medium is added to replace the volume. In such perfusion culturing methods, the removed cell culture is often filtered to retain the mammalian cells in the bioreactor for further recombinant protein production, while the culture medium (sometimes referred to as "spent medium") containing a recombinant protein is recovered.

Conventional methods and devices for filtering cell culture from a perfusion bioreactor have several disadvantages. For example, closed system alternating tangential flow filtration (ATF) results in the cell culture spending a long period of time outside the bioreactor controlled growth conditions (long external residence time), and traditional unidirectional tangential flow filtration (TFF), with no reverse flow, causes filter fouling. As such, conventional perfusion bioreactor methods often involve the cell culture spending a long period of time outside of the bioreactor controlled growth conditions, leading to decreases in viable cell density, percent viability, and culture specific and volumetric productivity. Further, previous methods often result in incomplete flushing of system filters leading to filter fouling.

Document US2011/0111486 discloses a bioreactor comprising: a container for holding a fluid culture; a first unidirectional valve; a second unidirectional valve and a diaphragm pump in fluid communication with the first and second valves, wherein the first unidirectional valve allows fluid culture flow away from the container and toward the diaphragm pump and the second unidirectional valve allows fluid culture flow towards the container and away from the diaphragm pump.

Document WO2014/051503 wich is considered as comprised in the state of the art pursuant to Article 54(3) EPC discloses a system for perfusion culture of cells comprising a) at least one bioreactor, b) at least one filter unit comprising a retentate inlet end, a retentate outlet end and at least one permeate outlet port, c) at least one reciprocating pump fluidically connected to the retentate inlet end, wherein said retentate inlet end is fluidically connected to said bioreactor via an inlet check valve arranged to allow flow in the direction from the bioreactor to the retentate inlet end and to block flow in the reverse direction, and wherein said retentate outlet end is fluidically connected to said bioreactor via an outlet check valve arranged to allow flow in the direction from the retentate outlet end to the bioreactor and to block flow in the reverse direction.

### SUMMARY

Applicants have discovered that an open circuit filtration system providing reversible tangential fluid flow across a surface of a cross-flow filter, as opposed to conventional unidirectional open circuit or bidirectional closed circuit filtration systems, provides for increased viable cell density, increased percentage viable cells, increased specific and/or volumetric productivity, increased specific glucose consumption, and decreased filter fouling. In the present disclosure the term reservoir is to be understood as bioreactor.

The open circuit filtration systems provided herein provide optimal conditions for recombinant protein production and yield, such as one or more of decreased external volume of cell culture (outside of the reservoir), increased exchange fraction (e.g., within the first conduit, the TFF unit, and the second conduit), decreased external residence time of cell culture (outside the reservoir), decreased sheer stress during cell culture filtration, improved cell viability in cell culture, elevated viable cell density in cell culture, and/or decreased filter fouling (due to better flushing of the filter(s)), e.g., as compared to other unidirectional open circuit filtration systems (e.g., unidirectional TFF systems) or bidirectional closed circuit filtration systems (closed circuit ATF™ systems). Accordingly, provided herein are open circuit filtration systems including a bioreactor, a tangential flow filtration (TFF) unit having first and second inlets, a first conduit in fluid communication between the reservoir and the TFF unit first inlet, and a second conduit in fluid communication between the reservoir and the TFF unit second inlet, and at least one pump disposed within the system, such that actuating the at least one pump flows fluid reversibly through the system from the bioreactor, through the first conduit, the TFF unit, the second conduit, and back to the bioreactor. Also provided are methods of processing a cell culture that include (a) providing an open circuit filtration system (e.g., any of the open circuit filtration systems described herein), (b) flowing cell culture from the reservoir through the TFF unit in a first flow direction for a first period of time, (c) reversing the first flow direction and flowing the cell culture through the TFF unit in a second flow direction for a second period of time, (d) reversing the second flow direction and flowing the culture through the TFF unit in the first flow direction for a third period of time, (e) repeating steps (c) - (d) at least two times, and (f) collecting the filtrate.

Provided herein are methods of processing a cell culture. These methods include the steps of: (a) providing an open circuit filtration system includes a reservoir comprising a cell culture, a tangential flow filtration (TFF) unit having first and second inlets, a first conduit in fluid communication between the reservoir and the TFF unit first inlet, and a second conduit in fluid communication between the reservoir and the TFF unit second inlet, and at least one pump disposed within the system for flowing fluid through the system, where the system is configured such that fluid can be flowed reversibly through the system from or to the reservoir and through the first and second conduits and the TFF unit via the at least one pump, and filtrate can be collected from the TFF unit; (b) flowing cell culture from the reservoir through the TFF unit in a first flow direction for a first period of time, (c) reversing the first flow direction and flowing the cell culture through the TFF unit in a second flow direction for a second period of time; (d) reversing the second flow direction and flowing the culture through the TFF unit in the first flow direction for a third period of time; (e) repeating steps (c) - (d) at least two times; and (f) collecting the filtrate. In some examples, the reservoir is a bioreactor or a refrigerated holding tank. In some examples, one or both of the first and second conduits include biocompatible tubing. The TFF unit can include a single cross-flow filter (e.g., a tubular cross-flow filter) or can include two or more cross-flow filters.

In some examples, the system includes one or more additional TFF units disposed in the first conduit, the second conduit, or both. In some examples, the cross-flow filter(s) have an average pore size of about 0.2 micrometer.

In some examples, the at least one pump is disposed in the first conduit or the second conduit, or both. In additional examples, the at least one pump is disposed in the system between any two TFF units. In some cases, the at least one pump is disposed in the reservoir and proximal to the first or second fluid conduit. In some embodiments of all the methods described herein, the at least one pump is a low turbulence pump (LTP) (e.g., a peristaltic pump). In some examples, the system includes a first and a second LTP, wherein the first LTP flows the cell culture in the first direction and the second LTP flows the cell culture in the second direction. In some embodiments, the system includes a single LTP, where the single LTP flows the cell culture in the first direction during the first and third time periods and flows the cell culture in the second direction during the second time period.

In any of the methods described herein, the first, second, and third periods of time are about 30 seconds to about 15 minutes. In some embodiments, the cell culture is flowed in one or more of (a), (b), and (c) at a rate of between about 0.5 L/minute and about 80 L/minute (e.g., between about 3.0 L/minute and about 60 L/minute).

The single repetition of (b) and (c) may result in an exchange fraction of greater than 50%. In some examples, the filtrate does not contain a mammalian cell. The cell culture may contain a secreted recombinant protein and the filtrate contains the secreted recombinant protein. The secreted recombinant protein may be an antibody or antigen-binding fragment thereof, a growth factor, a cytokine, or an enzyme, or a combination thereof. The method may further include a step of isolating the secreted recombinant protein from the filtrate. For example, the isolating can be performed using an integrated and continuous process that includes isolating through at least one multi-column chromatography system (MCCS). The method may further include a step of formulating a therapeutic drug substance by mixing the isolated recombinant protein with a pharmaceutically acceptable excipient or buffer. In some cases, the cell culture or filtrate, or both, are sterile. In some examples, the method is continuously performed for a period of between about 14 days to about 80 days.

Also provided are open circuit filtration systems that include a reservoir, a tangential flow filtration (TFF) unit having first and second inlets, a first conduit in fluid communication between the reservoir and the TFF unit first inlet, and a second conduit in fluid communication between the reservoir and the TFF unit second inlet, and at least one pump disposed within the system, where actuating the at least one pump flows fluid reversibly through the system from the reservoir, through the first conduit, the TFF unit, the second conduit, and back to the reservoir. The reservoir is a bioreactor. In some cases, one or both of the first and second conduits comprise(s) biocompatible tubing. In some cases, the TFF unit includes a single cross-flow filter (e.g., a tubular cross-flow filter). In some cases, the TFF unit includes two or more cross-flow filters. In some examples, the system includes one or more additional TFF units disposed in the first conduit, the second conduit, or both. In some systems, the cross-flow filter(s) have an average pore size of about 0.2 micrometer.

In some embodiments of the systems described herein, the at least one pump is disposed in the first conduit or the second conduit, or both. In some cases, the at least one pump is disposed in the system between any two TFF units. In other examples, the at least one pump is disposed in the reservoir and proximal to the first or second fluid conduit. In any of the systems described herein, the at least one pump is a low turbulence pump (LTP) (e.g., a peristaltic pump). In some embodiments, the system includes a first and a second LTP, where the first LTP is adapted to flow the cell culture in a first flow direction and the second LTP is adapted to reverse the first flow direction and flow the cell culture in a second flow direction. In other embodiments, the system includes a single LTP adapted to reversibly flow the cell culture in a first and second flow directions. In some cases, the peristaltic pump has a pump head volume of between about 20 mL and about 250 mL. The systems described herein may include a filtrate holding tank and a filtrate conduit in fluid communication between the TFF unit and the filtrate holding tank. Some systems described herein further include a biological manufacturing system comprising at least one multi-column chromatography system (MCCS) and an inlet and an outlet and a filtrate conduit in fluid communication between the TFF unit and the inlet of the biological manufacturing system, wherein the device is configured such that filtrate is passed into the inlet of the biological manufacturing system, through the at least one MCCS, and exits the device through the outlet of the biological manufacturing system. In any of the systems described herein, the TFF unit is disposed in a housing.

As used herein, the word "a" or "plurality" before a noun represents one or more of the particular noun. For example, the phrase "a mammalian cell" represents "one or more mammalian cells," and the phrase "plurality of microcarriers" means "one or more microcarriers."

The term "mammalian cell" means any cell from or derived from any mammal (e.g., a human, a hamster, a mouse, a green monkey, a rat, a pig, a cow, or a rabbit). In some embodiments, the mammalian cell can be, e.g., an immortalized cell, a differentiated cell, or an undifferentiated cell.

The term "cell culture" means a plurality of mammalian cells (e.g., any of the mammalian cells described herein) suspended in a liquid culture medium (e.g., any of the liquid culture media described herein). The cell culture can have a cell density of greater than about $0.1 \times 10^6$ cells/mL (e.g., greater than about $1 \times 10^6$ cells/mL, greater than about $5 \times 10^6$ cells/mL, greater than about $10 \times 10^6$ cells/mL, greater than about $15 \times 10^6$ cells/mL, greater than about $20 \times 10^6$ cells/mL, greater than about $25 \times 10^6$ cells/mL, greater than about $30 \times 10^6$ cells/mL, greater than about $35 \times 10^6$ cells/mL, greater than about $40 \times 10^6$ cells/mL, greater than about $45 \times 10^6$ cells/mL, greater than about $50 \times 10^6$ cells/mL, greater than about $55 \times 10^6$ cells/mL, greater than about $60 \times 10^6$ cells/mL, greater than about $65 \times 10^6$ cells/mL, greater than about $70 \times 10^6$ cells/mL, greater than about $75 \times 10^6$ cells/mL, greater than about $80 \times 10^6$ cells/mL, greater than about $85 \times 10^6$ cells/mL, greater than about $90 \times 10^6$ cells/mL, greater than about $95 \times 10^6$ cells/mL, or greater than $100 \times 10^6$ cells/mL). In some examples, the mammalian cells present in a cell culture are attached to microcarriers (e.g., any of the microcarriers described herein or known in the art).

The term "bioreactor" is art-known and means a vessel that can incubate a cell culture under a controlled set of physical conditions that allow for the maintenance or growth of a mammalian cell in a liquid culture medium. For example, the bioreactor can incubate a cell culture under conditions that allow for a mammalian cell in the cell culture to produce and secrete a recombinant protein. For example, a bioreactor typically includes an $O_2$ and $N_2$ sparge, a thermal jacket, one or more fluid ports, and an agitation system. Non-limiting examples of bioreactors are described herein. Additional examples of bioreactors are known in the art.

The term "open circuit filtration system" means a reservoir (e.g., a bioreactor) and a continuous closed fluid loop that both begins and ends at a reservoir, and includes a TFF unit through which a fluid (e.g., cell culture) in the closed fluid loop can pass to and from the reservoir (in either a first or second flow direction) through the TFF unit and back to the reservoir. The open circuit filtration system also includes at least one pump suitable for pumping the fluid (e.g., cell culture) to and/or from the reservoir through the TFF unit and back to the reservoir.

The terms "tangential flow filtration unit" or "TFF unit" are art-known and mean a device that includes at least one housing (such as a cylinder) and at least one cross-flow filter positioned in the housing such that a large portion of the filter's surface is positioned parallel to the flow of a fluid (e.g., a cell culture) through the unit. TFF units are well-known in the art and are commercially available. Exemplary commercially-available TFF units include Minimate™ TFF capsules (Pall Corporation), Vivaflow® 50 and 200 systems (Sartorius), BioCap 25, E0170, E0340, and E1020 capsules (3M), and ATF4 filter (Refine Technology). The housing can include a first inlet/outlet and a second inlet/outlet positioned, e.g., to allow fluid to pass through the first inlet/outlet, cross the at least one cross-flow filter, and through the second inlet/outlet. In some examples, an open-circuit filtration system can include multiple TFF units, e.g., connected in series and/or in parallel. For example, a system that includes two or more TFF units can include fluid conduits fluidly connecting neighboring pairs of TFF units in the system. In other examples, a system can include two or more sets of two or more TFF units fluidly connected by fluid conduits. Any of the TFF units described herein or known in the art are capable of receiving fluid in a first flow direction and a second flow direction.

The term "cross-flow filter" is art known and means a filter that designed such that it can be positioned in a TFF unit such that a large portion of the filter's surface is parallel to the flow (e.g., first and second flow direction) of a fluid (e.g., a cell culture). For example, a cross-flow filter can have any shape that allows for tangential flow filtration, e.g., a tubular or rectangular shape. Particularly useful cross-flow filters are designed to result in a low amount of fluid turbulence or sheer stress in the fluid (e.g., cell culture) when the fluid is flowed in a first and second direction across the surface of the cross-flow filter. Cross-flow filters are commercially available, e.g., from Sartorius, MembraPure, Millipore, and Pall Corporation.

The term "low turbulence pump" or "LTP" is art-known and means a device that can move a fluid (e.g., a cell culture) within the system in a single direction (e.g., a first or second flow direction) or reversibly flowing a fluid (e.g., a cell culture) in two directions (a first and second flow direction) within the system without inducing a substantial amount of sheer stress or fluid turbulence in the fluid (e.g., cell culture). When a LTP is used to flow a fluid (e.g., a cell culture) in alternating first and second flow directions, the second flow direction is approximately opposite to that of the first flow direction. An example of a LTP is a peristaltic pump. Other examples of LTPs are known in the art.

The terms "reversing the flow" or "reversing the flow direction" are well-known to those of skill in the art. For example, skilled practicioners will appreciate that reversing the flow of a fluid means changing the overall flow direction of a fluid to a generally opposite overall flow direction (e.g., flow direction of a cell culture in any of the methods or systems described herein).

The term "exchange fraction" means the percentage of fluid (e.g., cell culture) that is returned to the reservoir after flowing the fluid through the components of an open circuit filtration system outside of the reservoir (e.g., through the first conduit, the at least one TFF unit, and the second conduit) in a first direction for a first period of time and flowing the fluid in a second direction for a second period of time.

The term "substantially free" means a composition (e.g., a liquid or solid) that is at least or about 90% free (e.g., at least or about 95%, 96%, 97%, 98%, or at least or about 99% free, or about 100% free) of a specific substance (e.g., a mammalian cell or host mammalian cell protein or nucleic acid). For example, a filtrate generated using the methods described herein can be substantially free of a mammalian cell or a microcarrier. In another example, a recombinant protein isolated using any of the processes described herein can be substantially free of a host mammalian cell protein, nucleic acid, and/or a contaminating virus.

The term "culturing" or "cell culturing" means the maintenance or growth of a mammalian cell in a liquid culture medium under a controlled set of physical conditions.

The term "liquid culture medium" means a fluid that contains sufficient nutrients to allow a mammalian cell to grow in the medium *in vitro*. For example, a liquid culture medium can contain one or more of: amino acids (e.g., 20 amino acids), a purine (e.g., hypoxanthine), a pyrimidine (e.g., thymidine), choline, inositol, thiamine, folic acid, biotin, calcium, niacinamide, pyridoxine, riboflavin, thymidine, cyanocobalamin, pyruvate, lipoic acid, magnesium, glucose, sodium, potassium, iron, copper, zinc, selenium, and other necessary trace metals, and sodium bicarbonate. A liquid culture medium may contain serum from a mammal. In some instances, a liquid culture medium does not contain serum or another extract from a mammal (a defined liquid culture medium). A liquid culture medium may contain trace metals, a mammalian growth hormone, and/or a mammalian growth factor. Non-limiting examples of liquid culture medium are described herein and additional examples are known in the art and are commercially available.

The term "microcarrier" means a particle (e.g., an organic polymer) that has a size of between 20 μm to about 1000 μm that contains a surface that is permissive or promotes attachment of a mammalian cell (e.g., any of the mammalian cells described herein or known in the art). A microcarrier can contain one or more pores (e.g., pores with an average diameter of about 10 μm to about 100 μm). Non-limiting examples of microcarriers are described herein. Additional examples of microcarriers are known in the art. A microcarrier can contain, e.g., a polymer (e.g., cellulose, polyethylene glycol, or poly-(lactic-co-glycolic acid)).

The term "animal-derived component free liquid culture medium" means a liquid culture medium that does not contain any components (e.g., proteins or serum) derived from an animal.

The term "serum-free liquid culture medium" means a liquid culture medium that does not contain animal serum.

The term "serum-containing liquid culture medium" means a liquid culture medium that contains animal serum.

The term "chemically-defined liquid culture medium" means a liquid culture medium in which substantially all of the chemical components are known. For example, a chemically-defined liquid culture medium does not contain fetal bovine serum, bovine serum albumin, or human serum albumin, as these preparations typically contain a complex mix of albumins and lipids.

The term "protein-free liquid culture medium" means a liquid culture medium that does not contain any protein (e.g., any detectable protein).

The term "immunoglobulin" means a polypeptide containing an amino acid sequence of at least 15 amino acids (e.g., at least 20, 30, 40, 50, 60, 70, 80, 90, or 100 amino acids) of an immunoglobulin protein (e.g., a variable domain sequence, a framework sequence, or a constant domain sequence). The immunoglobulin may, for example, include at least 15 amino acids of a light chain immunoglobulin and/or at least 15 amino acids of a heavy chain immunoglobulin. The immunoglobulin may be an isolated antibody (e.g., an IgG, IgE, IgD, IgA, or IgM). The immunoglobulin may be a subclass of IgG (e.g., IgG1, IgG2, IgG3, or IgG4). The immunoglobulin may be an antibody fragment, e.g., a Fab fragment, a F(ab')$_2$ fragment, or a scFv fragment. The immunoglobulin may also be a bi-specific antibody or a tri-specific antibody, or a dimer, trimer, or multimer antibody, or a diabody, an Affibody®, or a Nanobody®. The immunoglobulin can also be an engineered protein containing at least one immunoglobulin domain (e.g., a fusion protein). Non-limiting examples of immunoglobulins are described herein and additional examples of immunoglobulins are known in the art.

The term "protein fragment" or "polypeptide fragment" means a portion of a polypeptide sequence that is at least or about 4 amino acids, e.g., at least or about 5 amino acids, at least or about 6 amino acids, at least or about 7 amino acids, at least or about 8 amino acids, at least or about 9 amino acids, at least or about 10 amino acids, at least or about 11 amino acids, at least or about 12 amino acids, at least or about 13 amino acids, at least or about 14 amino acids, at least or about 15 amino acids, at least or about 16 amino acids, at least or about 17 amino acids, at least or about 18 amino acids, at least or about 19 amino acids, or at least or about 20 amino acids in length, or more than 20 amino acids in length. A recombinant protein fragment can be produced using any of the methods described herein.

The term "engineered protein" means a polypeptide that is not naturally encoded by an endogenous nucleic acid present within an organism (e.g., a mammal). Examples of engineered proteins include enzymes (e.g., with one or more amino acid substitutions, deletions, insertions, or additions that result in an increase in stability and/or catalytic activity of the engineered enzyme), fusion proteins, antibodies (e.g., divalent antibodies, trivalent antibodies, or a diabody), and antigen-binding proteins that contain at least one recombinant scaffolding sequence.

The term "isolate" or "isolating" in certain contexts means at least partially purifying or purifying (e.g., at least or about 5%, e.g., at least or about 10%, 15%, 20%, 25%, 30%, 40%, 45%, 50%, 55%, 60%, 65%, 70%, 75%, 80%, 85%, 90%, or at least or about 95% pure by weight) a recombinant protein from one or more other components present in the filtrate (e.g., a filtrate generated using the presently described methods), for example one or more components of DNA, RNA, and/or other proteins present in the filtrate. Non-limiting methods for isolating a protein from a filtrate are described herein and others are known in the art.

The term "secreted protein" or "secreted recombinant protein" means a protein or a recombinant protein that originally contained at least one secretion signal sequence when it is translated within a mammalian cell, and through, at least in part, enzymatic cleavage of the secretion signal sequence in the mammalian cell, is released at least partially into the extracellular space (e.g., a liquid culture medium).

The phrase "gradient perfusion" is art-known and refers to the incremental change (e.g., increase or decrease) in the volume of culture medium removed and added to an initial culture volume over incremental periods (e.g., an about 24-hour period, a period of between about 1 minute and about 24-hours, or a period of greater than 24 hours) during the culturing period (e.g., the culture medium re-feed rate on a daily basis). The fraction of media removed and replaced each day can vary depending on the particular cells being cultured, the initial seeding density, and the cell density at a particular time.

"Specific productivity rate" or "SPR" as used herein refers to the mass or enzymatic activity of a recombinant protein produced per mammalian cell per day. The SPR for a recombinant antibody is usually measured as mass/cell/day. The SPR for a recombinant enzyme is usually measured as units/cell/day or (units/mass)/cell/day.

"Volume productivity rate" or "VPR" as used herein refers to the mass or enzymatic activity of recombinant protein produced per volume of culture (e.g., per L of bioreactor, vessel, or tube volume) per day. The VPR for a recombinant antibody is usually measured as mass/L/day. The VPR for a recombinant enzyme is usually measured as units/L/day or mass/L/day.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

Other features and advantages of the invention will be apparent from the following detailed description and figures, and from the claims.

**DESCRIPTION OF DRAWINGS**

Figure 1 is a schematic diagram showing an exemplary open circuit filtration system that can be used to process a cell culture. The system shown includes a single pump 8 disposed in a first conduit **6.**

Figure 2 is a schematic diagram showing an exemplary open circuit filtration system that includes a single pump **8** disposed in a second conduit **7.**

Figure 3 is a schematic diagram showing an exemplary open circuit filtration system that includes a single pump **8** disposed in a reservoir **2** (e.g., a bioreactor) and proximal to a first conduit **6.**

Figure 4 is a schematic diagram showing an exemplary open circuit filtration system that includes a two TFF units **3** that each include two cross-flow filters **12,** where the two TFF units **3** are fluidly connected by a third conduit **14,** and a single pump **8** is disposed in the third conduit **14.**

Figure 5 is a schematic diagram showing an exemplary open circuit filtration system that includes a single pump **8** disposed in a second conduit **7,** and includes several pressure sensors **14,** and a flowmeter **15.**

Figure 6 is a schematic diagram showing an exemplary open circuit filtration system that includes a pump **8** disposed in the first conduit **6** and a pump **8** disposed in a second conduit **7.**

Figure 7 is a schematic diagram showing an exemplary open circuit filtration system that includes a reservoir **2** and a first and second subsystems **19.**

Figure 8 is a schematic diagram showing the first flow direction in an exemplary system.

Figure 9 is a diagram showing the flow of a cell culture over a first period of time in a first flow direction, a reversal of the first flow direction over a time period ($t_{r1}$), flow of the cell culture over a second period of time in a second

flow direction ($t_2$), reversal of the second flow direction over a time period ($t_{r2}$), and flowing the cell culture for a third period of time in the first flow direction ($t_3$). In the diagram, F represents the cell culture flow rate (L/minute).

Figure 10 is a graph of the viable cell density in a cell culture processed using the methods provided herein (GC2008 Set6 TFF V24; gray) or using ATF™ (Refine Technology) filtration (GC2008 Set5 ATF™ V21; black).

Figure 11 is a graph of the percent viable cells in a cell culture processed using the methods provided herein (gray) or using ATF™ (Refine Technology) filtration (black).

Figure 12 is a graph of the capacitance (pF) of cell culture processed using the methods provided herein (gray) or using ATF™ (Refine Technology) filtration (black).

Figure 13 is a graph of the mean viable cell diameter of cell culture processed using methods provided herein (gray) or using ATF™ (Refine Technology) filtration (black).

Figure 14 is a graph of the secreted immunoglobulin (IgG) detected in cell culture processed using methods provided herein (gray) and using ATF™ (Refine Technology) filtration (black).

Figure 15 is a graph of the volumetric productivity (g/L/d) of cell culture processed using methods provided herein (gray) and using ATF™ (Refine Technology) filtration (black).

Figure 16 is a graph of the specific productivity (pg/cell/day) of cell culture processed using methods provided herein (gray) and using ATF™ (Refine Technology) filtration (black).

Figure 17 is a graph of the percentage sieving coefficient of cell culture processed using methods provided herein (gray) or using ATF™ (Refine Technology) filtration (black).

Figure 18 is a graph of the specific glucose consumption (ng/cell/day) of cell culture processed using methods provided herein (GC2008 Set6 TFF V24) (gray) or using ATF™ (Refine Technology) filtration (black).

Figure 19 is a graph of the specific lactate production (ng/cell/day) of cell culture processed using methods provided herein (gray) or using ATF™ (Refine Technology) filtration (black).

Figure 20 is a graph of the specific aerobic glucose consumption (cpmol/cell/hour) of cell culture processed using methods described herein (gray) or using ATF™ (Refine Technology) filtration (black).

Figure 21 is a graph of the lactate yield from glucose (mol/mol) of cell culture processed using methods described herein (gray) or using ATF™ (Refine Technology) filtration (black).

## DETAILED DESCRIPTION

Provided herein are open circuit filtration systems that include a reservoir, understood as a bioreactor in the present disclosure, a TFF unit having first and second inlets, a first conduit in fluid communication between the reservoir and the TFF unit first inlet, and a second conduit in fluid communication between the reservoir and the TFF unit second inlet, and at least one pump disposed within the system, wherein actuating the at least one pump flows fluid reversibly through the system from the reservoir, through the fluid conduit, the TFF unit, the second conduit, and back to the reservoir. Also provided are methods of processing a cell culture that includes using an open circuit filtration system (e.g., any of the open circuit filtration systems described herein). The systems and methods described herein provide, for example, high cell viability and/or percentage cell viability during cell culture processing. Additional benefits of the systems and methods provided herein are described below.

### Open Circuit Filtration Systems

The present specification provides exemplary open circuit filtration systems useful for performing the methods described herein. These systems are designed such that actuating at least one pump (in the system) flows fluid reversibly through the system from the reservoir, through the first conduit, the TFF unit, the second conduit, and back to the reservoir.

### *Exemplary Single Pump Systems*

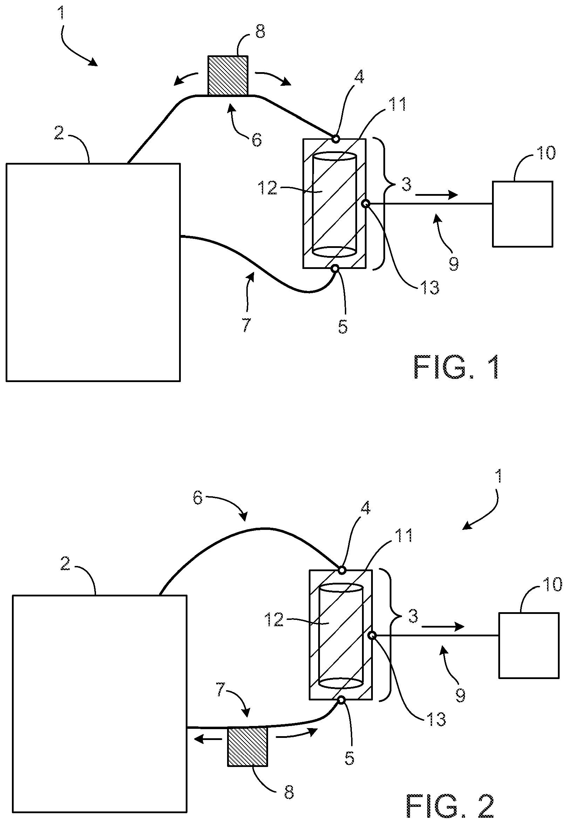

A non-limiting example of a system **1** is provided in FIG. 1. System **1** includes a reservoir **2**, e.g., a bioreactor, a first conduit **6**, a second conduit **7**, and a TFF unit **3** that includes a housing **11** and a single cross-flow tubular filter **12**, a first inlet **4**, and a second inlet **5**. The single cross-flow tubular filter **12** can have a pore size, e.g., of about 0.2 μm. The first conduit **6** is in fluid communication between the reservoir **2** and the first inlet **4**. The second conduit **7** is in fluid communication between the reservoir **2** and the second inlet **5**. Fluid conduit **6** and fluid conduit **7** can be any type of biocompatible tubing, e.g., silicone tubing. The TFF unit **3** can include a single cross-flow tubular filter **12**, as shown in FIG. 1, or two or more cross-flow filters.

System **1** in FIG. 1 also includes a pump **8**, e.g., a low turbulence pump (LTP), such as a peristaltic pump, that is disposed in the first conduit **6**. When actuated, the pump **8** flows fluid reversibly through the system from the reservoir **2**, through the first conduit **6**, the TFF unit **3**, the second conduit **7**, and back to the reservoir **2**. The housing **11** of the TFF unit **3** includes a filtrate outlet **13**. System **1** also includes a filtrate holding tank **10** and a filtrate conduit **9** in fluid communication between the filtrate outlet **13** and the filtrate holding tank **10**. The filtrate holding tank **10** can be, e.g., a refrigerated holding tank. The filtrate conduit **9** can be any type of biocompatible tubing, e.g., silicone tubing.

Another exemplary system **1** is shown in FIG 2, which is similar to that shown in FIG. 1, except at least that the LTP is situated in a different portion of the system. System **1** includes a reservoir **2**, e.g., a bioreactor, a first conduit **6,** a second conduit 7, and a TFF unit **3** that includes a housing **11** and a single cross-flow tubular filter **12,** a first inlet **4,** and a second inlet **5**. The single cross-flow tubular filter **12** can have a pore size, e.g., of about 0.2 $\mu$m. The first conduit **6** is in fluid communication between the reservoir **2** and the first inlet **4**. The second conduit **7** is in fluid communication between the reservoir **2** and the second inlet **5**. Fluid conduit **6** and fluid conduit **7** can be any type of biocompatible tubing, e.g., silicone tubing. The TFF unit **3** can include a single cross-flow tubular filter **12,** as shown in FIG. 2, or can include a set of two or more cross-flow filters.

System **1** in FIG. 2 also includes a pump **8**, e.g., a low turbulence pump (LTP), such as a peristaltic pump, that is disposed in the second conduit **7**. When actuated, the pump **8** flows fluid reversibly through the system from the reservoir **2**, through the first conduit **6,** the TFF unit **3**, the second conduit **7**, and back to the reservoir **2**. The housing **11** of the TFF unit **3** includes a filtrate outlet **13**. System **1** also includes a filtrate holding tank **10** and a filtrate conduit **9** in fluid communication between the filtrate outlet **13** and the filtrate holding tank **10**. The filtrate holding tank **10** can be, e.g., a refrigerated holding tank. The filtrate conduit **9** can be any type of biocompatible tubing, e.g., silicone tubing.

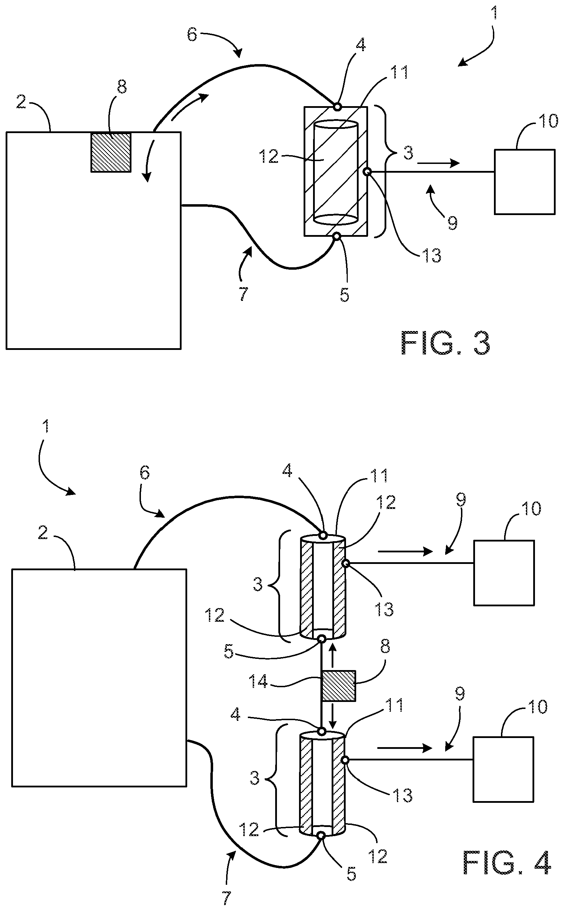

An additional exemplary system **1** is shown in FIG 3. System **1** includes a reservoir **2**, e.g., a bioreactor, a first conduit **6**, a second conduit **7,** and a TFF unit **3** that includes a housing **11** and a single cross-flow tubular filter **12,** a first inlet **4,** and a second inlet **5.** The single cross-flow tubular filter **12** can have a pore size, e.g., of about 0.2 $\mu$m. The first conduit **6** is in fluid communication between the reservoir **2** and the first inlet **4**. The second conduit **7** is in fluid communication between the reservoir **2** and the second inlet **5**. Fluid conduit **6** and fluid conduit **7** can be any type of biocompatible tubing, e.g., silicone tubing. The TFF unit **3** can include a single cross-flow tubular filter **12,** as shown in FIG. 3, or can contain a set of two or more cross-flow filters.

System **1** in FIG. 3 also includes a single pump **8**, e.g., a low turbulence pump (LTP), such as a peristaltic pump, that is disposed in the reservoir **2**, e.g., bioreactor, and proximal to the first conduit **6**. When actuated, the single pump **8** flows fluid reversibly through the system from the reservoir **2**, through the first conduit **6,** the TFF unit **3**, the second conduit **7,** and back to the reservoir **2**. The housing **11** of the TFF unit **3** includes a filtrate outlet **13**. System **1** also includes a filtrate holding tank **10** and a filtrate conduit **9** in fluid communication between the filtrate outlet **13** and the filtrate holding tank **10**. The filtrate holding tank **10** can be, e.g., a refrigerated holding tank. The filtrate conduit **9** can be any type of biocompatible tubing, e.g., silicone tubing.

Exemplary system **1** is shown in FIG. 4, which is similar to those illustrated in FIGS. 1-3, except at least the system includes multiple TFF units. System **1** includes a reservoir **2**, e.g., a bioreactor, a first conduit **6,** a second conduit **7,** and two TFF units **3** that each include: a housing **11,** a first inlet **4,** a second inlet **5,** and two cross-flow filters **12**. The two TFF units **3** are fluidly connected by a third conduit **14**. Each of the cross-flow filters **12** can have a pore size, e.g., of about 0.2 $\mu$m. The first conduit **6** is in fluid communication between the reservoir **2** and the first inlet **4** of one of the two TFF units **3,** and the second conduit **7** is in fluid communication between the reservoir **2** and the second inlet **5** of the other of the two TFF units 3. The third conduit is in fluid communication between the second inlet **5 of a** TFF unit **3** and the first inlet **4** of the other TFF unit **3,** as shown, e.g., in FIG. 4. Fluid conduits **6, 7,** and **14** can be any type of biocompatible tubing, e.g., silicone tubing. As can be appreciated by those in the art, the TFF units **3** can alternatively contain a single cross-flow filter, e.g., a tubular cross-flow filter.

System **1** in FIG. 4 also includes a single pump **8**, e.g., a low turbulence pump (LTP), such as a peristaltic pump, that is disposed in the third conduit **14**. When actuated, the single pump **8** flows fluid reversibly through the system from the reservoir **2**, through the first conduit **6,** a TFF unit **3**, the third conduit **14,** the other TFF unit **3,** the second conduit 7, and back to the reservoir **2**. The housing **11** of each of the two TFF units **3** includes a filtrate outlet **13**. System **1** also includes two filtrate holding tanks **10** and two filtrate conduits **9**. Each single filtrate holding tank **10** is fluidly connected to a filtrate outlet **13** in a TFF unit **3** by a filtrate conduit **9**. The filtrate holding tanks **10** can be, e.g., a refrigerated holding tank. The filtrate conduits **9** can be any type of biocompatible tubing, e.g., silicone tubing.

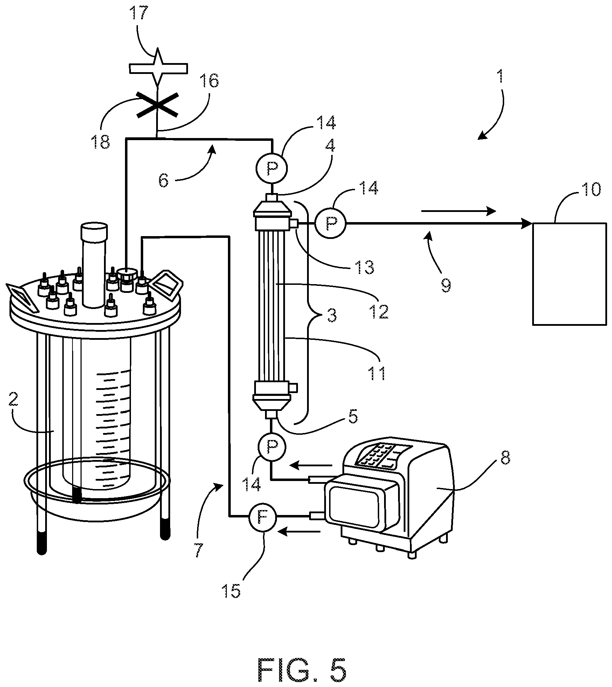

An additional exemplary system **1** is shown in FIG. 5. System **1** includes a reservoir **2**, e.g., a bioreactor, a first conduit **6,** a second conduit **7,** and a TFF unit **3** that includes a housing **11** and a single cross-flow tubular filter **12,** a first inlet **4,** and a second inlet **5**. The cross-flow filter **12** can have, e.g., a pore size of about 0.2 $\mu$m, a fiber count of about 830 fibers/filter, include fibers with an ID of 1 mm and a length of 30 cm, and have a filtration area of 0.77 m$^2$. The first conduit **6** is in fluid communication between the reservoir **2** and the first inlet **4**. The second conduit 7 is in fluid communication between the reservoir **2** and the second inlet **5**. Fluid conduits **6** and **7** can be any type of biocompatible tubing, e.g., silicone tubing. Fluid conduits **6** and **7** can be 0.5 inch internal diameter (ID) transfer tubing.

System **1** in FIG. 5 also includes a single pump **8**, e.g., a low turbulence pump (LTP), such as a peristaltic pump, that is disposed in the second conduit **7**. The pump **8** can be a Watson-Marlow peristaltic pump 620 Du equipped with twin channel GORE Sta-Pure tubing (16 mm ID, 4 mm wall). When actuated, the single pump **8** flows fluid reversibly through the system from the reservoir **2**, through the first conduit **6,** the TFF unit **3**, the second conduit **7,** and back to the reservoir **2**. The housing **11** of the TFF unit **3** includes a filtrate outlet **13**. System **1** also includes a filtrate holding tank **10** and a filtrate conduit **9** in fluid communication between the filtrate outlet **13** and the filtrate holding tank **10**. The filtrate holding tank **10** can be, e.g., a refrigerated holding tank. The filtrate conduit **9** can be any type of biocompatible tubing, e.g., silicone tubing. System **1** also includes a pressure sensors **14** disposed in each of the first conduit **6**, the filtrate conduit **9**, and the second conduit **7**. The pressure sensors **14** can be PendoTECH PressureMAT™ pressure sensors. System **1** also includes a flowmeter **15** disposed in the second conduit **7**. The flowmeter **15** can be a EM-TEC BioProTT, non-invasive, real-time flowmeter.

System **1** in FIG. 5 also includes a port conduit **16** and a port **17**, where the port conduit **16** is in fluid communication between the first conduit **6** and the port **17**. System **1** can also include a clamp **18** disposed in the port conduit **16**. The port **17** and the port conduit **16** can be used to add fluids into the system **1** through the first conduit **6**.

### *Exemplary Multiple Pump Systems*

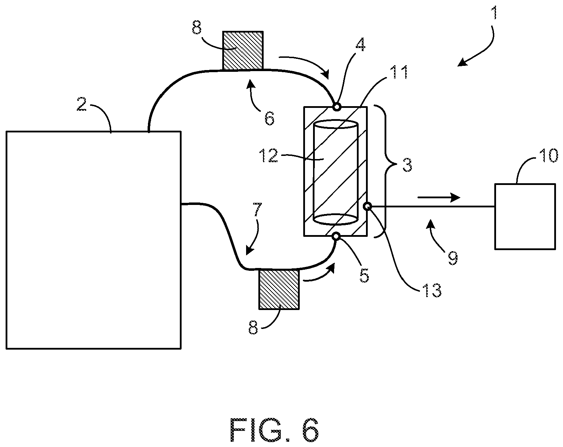

A non-limiting example of a system **1** including two pumps **8** is shown in FIG. 6. System **1** includes a reservoir **2**, e.g., a bioreactor, a first conduit **6**, a second conduit **7**, and a TFF unit **3** that includes a housing **11** and a single cross-flow tubular filter **12**, a first inlet **4**, and a second inlet **5**. The single cross-flow tubular filter **12** can have a pore size of about 0.2 $\mu$m. The first conduit **6** is in fluid communication between the reservoir **2** and the first inlet **4**. The second conduit **7** is in fluid communication between the reservoir **2** and the second inlet **5**. Fluid conduits **6** and **7** can be any type of biocompatible tubing, e.g., silicone tubing. The TFF unit **3** can include a single cross-flow tubular filter **12**, as shown in FIG. 6, or can include a set of two or more cross-flow filters.

System **1** in FIG. 6 also includes a pump **8**, e.g., a low turbulence pump (LTP), such as a peristaltic pump, that is disposed in the first conduit **6**, and a pump **8**, a low turbulence pump (LTP), such as a peristaltic pump, that is disposed in the second conduit **7**. When actuated, the pump **8** disposed in the first conduit **6** flows fluid in a first direction through the system from the reservoir **2**, through the first conduit **6**, the TFF unit **3**, the second conduit **7**, and back to the reservoir **2**. When actuated, the pump **8** disposed in the second conduit **7** flows fluid in a second direction (opposite to that of the first direction) through the system from the reservoir **2**, through the second conduit **7**, the TFF unit **3**, the first conduit **6**, and back to the reservoir **2**. The housing **11** of the TFF unit **3** includes a filtrate outlet **13**. System **1** also includes a filtrate holding tank **10** and a filtrate conduit **9** in fluid communication between the filtrate outlet **13** and the filtrate holding tank **10**. The filtrate holding tank **10** can be, e.g., a refrigerated holding tank. The filtrate conduit **9** can be any type of biocompatible tubing, e.g., silicone tubing.

### *Exemplary Systems that include Two or More Subsystems*

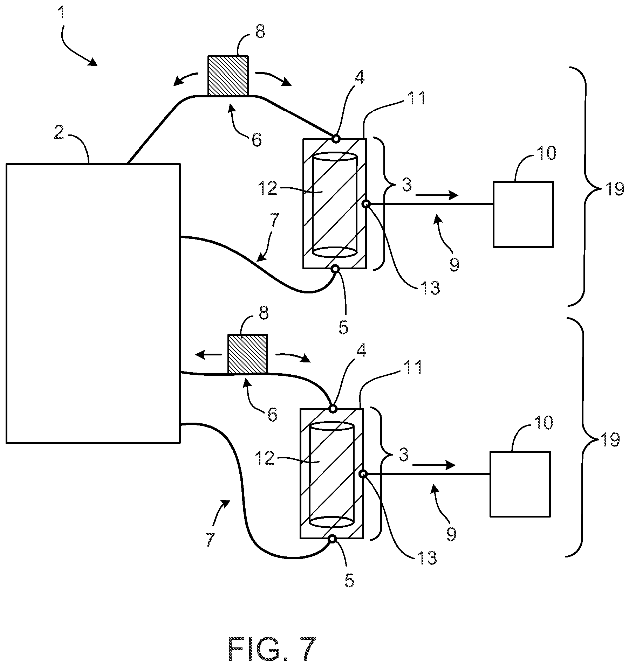

Skilled practicioners will appreciate that multiple subsystems can be added to the system. An exemplary system **1** including two or more subsystems **19** is shown in FIG. 7. System **1** includes a reservoir **2**; and a first and second subsystem **19**, each subsystem **19** including a first conduit **6**, a second conduit **7**, and a TFF unit **3** that includes a housing **11** and a single cross-flow tubular filter **12**, a first inlet **4**, and a second inlet **5**, as shown in FIG. 7. The single cross-flow tubular filters **12** can have a pore size of about 0.2 $\mu$m. In each subsystem, the first conduit **6** is in fluid communication between the reservoir **2** and the first inlet **4**. The second conduit **7**, in each subsystem, is in fluid communication between the reservoir **2** and the second inlet **5**. Fluid conduits **6** and **7** can be any type of biocompatible tubing, e.g., silicone tubing. The TFF units **3** can include a single cross-flow tubular filter **12**, respectively, as shown in FIG. 7, or can each include a set of two or more cross-flow filters. The single cross-flow tubular filters **12** can have a pore size of about 0.2 $\mu$m.

Each subsystem **19** in FIG. 7 also includes a single pump **8**, e.g., a low turbulence pump (LTP), such as a peristaltic pump, that is disposed in the first conduit **6**. When actuated, the single pump **8** in each subsystem **19** flows fluid reversibly through the system from the reservoir **2**, through the first conduit **6**, the TFF unit **3**, the second conduit **7**, and back to the reservoir **2**. The housing **11** of each of the two TFF units **3** includes a filtrate outlet **13**. Each subsystem **19** also includes a filtrate holding tank **10** and a filtrate conduit **9** in fluid communication between the TFF unit **3** and the filtrate holding tank **10**. The filtrate holding tanks **10** can be, e.g., a refrigerated holding tank. The filtrate conduits **9** can be any type of biocompatible tubing, e.g., silicone tubing.

### *Additional Exemplary System Structures and Features*

Non-limiting exemplary structures that can be used for the reservoir, the conduits, the TFF unit(s), the pump(s), the filtrate holding tank(s), flowmeter(s), pressure sensor(s), clamp(s), port(s), and biological manufacturing system(s) are described below.

*Reservoirs*

A reservoir can be a bioreactor. The bioreactor can have a volume of, e.g., between about 1 L to about 10,000 L (e.g., between about 1 L to about 50 L, between about 50 L to about 500 L, between about 500 L to about 1000 L, between 500 L to about 5000L, between about 500 L to about 10,000 L, between about 5000 L to about 10,000 L, between about 1 L and about 10,000 L, between about 1L and about 8,000 L, between about 1 L and about 6,000 L, between about 1 L and about 5,000 L, between about 100 L and about 5,000 L, between about 10 L and about 100 L, between about 10 L and about 4,000 L, between about 10 L and about 3,000 L, between about 10 L and about 2,000 L, or between about 10 L and about 1,000 L). Any of the bioreactors described herein can be a perfusion bioreactor. Exemplary bioreactors can be purchased from a number of different commercial vendors (e.g., Xcellerex (Marlborough, MA) and Holland Applied Technologies (Burr Ridge, IL)).

Alternatively or in addition, a reservoir can be a holding tank. For example, such a refrigerated holding tank can hold cell culture containing a recombinant protein for a period of between about 5 minutes and about one week (e.g., between about 5 minutes and about 6 days, between about 5 minutes and about 5 days, between about 5 minutes and about 4 days, between about 5 minutes and about 3 days, between about 5 minutes and about 2 days, between about 5 minutes and about 36 hours, between about 5 minutes and about 24 hours, between about 5 minutes and about 12 hours). The cell culture in the holding tank can be held at a temperature of between about 15 °C and about 37 ° C, between about 20° C and about 37° C, between about 25° C and about 37 ° C, between about 30° C and about 37° C, or between about 20° C and about 30° C.

*Conduits*

A conduit described herein can be simple tubing, e.g., biocompatible tubing. Non-limiting examples of useful tubing include silicone rubber, polyurethane, polydioxanone (PDO), polyhydroxyalkanoate, polyhydroxybutyrate, poly(glycerol sebacate), polyglycolide, polylactide, polycaprolactone, or polyanhydride, or copolymers or derivatives including these and/or other polymers. Alternatively or in addition, any of the conduits described herein can include polyvinyl chloride. Any of the conduits can have, for example, an inner diameter (ID) of between about between about 5 mm and about 50 mm (e.g., between about 10 mm about 40 mm, between about 10 mm and about 35 mm, or between about 10 mm and about 30 mm, between about 10 mm and about 20 mm). A conduit can be weldable transfer tubing. Additional examples of conduits and properties of conduits that can be used in the present devices and methods are well-known by those in the art.

*TFF Units and Cross-Flow Filters*

The TFF units used in any of the systems or subsystems, or methods described herein can include one or more cross-flow filters. For example, a TFF unit described herein can include a single cross-flow filter (e.g., a tubular cross-flow filter). In other examples, a TFF unit can include two or more (e.g., three, four, five, or six) cross-flow filters (e.g., tubular cross-flow filters). The two or more cross-flow filters in the TFF unit can be identical or can be different (e.g., different in number, type, shape, surface area, or pore size). In a specific example, the TFF unit can include two tubular cross-flow filters. The two or more cross-flow filters present in a TFF unit can be curved rectangular in shape.

The cross-flow filter(s) can have an average pore size of between about 0.1 $\mu$m to about 0.45 $\mu$m (e.g., between about 0.15 $\mu$m to about 0.40 $\mu$m, between about 0.15 $\mu$m to about 0.35 $\mu$m, between about 0.15 $\mu$m to about 0.30 $\mu$m, between about 0.15 $\mu$m to about 0.25 $\mu$m), or of about 0.20 $\mu$m. The cross-flow filter(s) can be a spectrum filter composed of polyethersulfone (PES).

The cross-flow filter(s) can have a surface area (filtration area) of between about 0.1 $m^2$ to about 5 $m^2$ (e.g., between about 0.5 $m^2$ to about 4.5 $m^2$, between about 0.5 $m^2$ to about 4.0 $m^2$, between about 0.5 $m^2$ to about 3.5 $m^2$, between about 0.5 $m^2$ to about 3.0 $m^2$, between about 0.5 $m^2$ to about 2.5 $m^2$, between about 0.5 $m^2$ to about 2.0 $m^2$, between about 0.5 $m^2$ to about 1.5 $m^2$, or between about 0.5 $m^2$ to about 1.0 $m^2$). The cross-flow filters can have a total numbers of fiber per filter of between about 500 fibers/filter to about 2500 fibers/filter (e.g., between about 500 fibers/filter to about 2400 fibers/filter, between about 500 fibers/filter to about 2300 fibers/filter, between about 500 fibers/filter to about 2200 fibers/filter, between about 500 fibers/filter to about 2100 fibers/filter, between about 500 fibers/filter to about 2000 fibers/filter, between about 500 fibers/filter to about 1900 fibers/filter, between about 500 fibers/filter to about 1800 fibers/filter, between about 500 fibers/filter to about 1700 fibers/filter, between about 500 fibers/filter to about 1600 fibers/filter, between about 500 fibers/filter to about 1500 fibers/filter, between about 500 fibers/filter to about 1400 fibers/filter, between about 500 fibers/filter to about 1300 fibers/filter, between about 500 fibers/filter to about 1200 fibers/filter, between about 500 fibers/filter to about 1100 fibers/filter, between about 500 fibers/filter to about 1000 fibers/filter, between about 500 fibers/filter to about 900 fibers/filter, between about 600 fibers/filter to about 900 fibers/filter, between about 700 fibers/filter to about 900 fibers/filter, or between about 800 fibers/filter to about 900 fibers/filter). In some examples, the fibers within the cross-flow filter(s) have an internal diameter of between about 0.05 mm to about 10 mm (e.g., between about 0.1 mm to about 9 mm, between about 0.1 mm to about 8 mm, between about 0.1 mm to about 7 mm, between about 0.1 mm to about 6 mm, between about 0.1 mm to about 5 mm, between about 0.1 mm to about 4 mm, between about 0.1 mm to about 3 mm, between about 0.1 mm to about 2.5 mm, between about 0.1 mm to about 2.0 mm, between about 0.1 mm to about 1.5 mm, between about 0.5 mm to about 1.5 mm, or between about 0.75 mm to about 1.25 mm). The fibers present in the cross-flow filter(s) can have a length of between about 0.2 cm and about 200 cm (e.g., between about 0.2 cm and about 190 cm, between about 0.2 cm and about 180 cm, between about 0.2 cm and about 170 cm, between about 0.2 cm and about 160 cm, between about 0.2 cm and about 150 cm, between about 0.2 cm and about 140 cm, between about 0.2 cm and about 130 cm, between about 0.2 cm and about 120 cm, between about 0.2 cm and about 110 cm, between about 0.2 cm and about 100 cm, between about 0.2 cm and about 90 cm, between about 0.2 cm and about 80 cm, between about 0.2 cm and about 70 cm, between about 0.2 cm and about 60 cm, between about 0.2 cm and about 55 cm, between about 0.2 cm and about 50 cm, between about 1 cm and about 45 cm, between about 1 cm and about 40 cm, between about 1 cm and about 35 cm, between about 1 cm and about 35 cm, between about 1 cm and about 30 cm, between about 1 cm and about 25 cm, between about 1 cm and about 20 cm, between about 1 cm and about 15 cm, between about 1 cm and about 10 cm, between about 0.1 cm and about 5 cm, between about 20 cm and about 40 cm, or between about 25 cm and about 35 cm). The cross-flow filter(s) can have any shape, such that the majority of the surface area of the filter(s) is positioned parallel to the flow of the fluid (e.g., cell culture) in the system. For example, the cross-flow filter(s) can have a tubular shape or a curved rectangular or donut-shape. An example of a cross-flow filter than can be used in systems described herein is the ATF4 filter (Refine Technology). Additional cross-flow filters are described herein and are known in the art.

As can be appreciated by those skilled in the art, the cross-flow filter(s) in the TFF unit can be housed in a casing (e.g., hard plastic or metal casing). A housing can be in any shape, cylindrical or rectangular, and designed such that it can hold one or more cross-flow filters. The housing can contain a surface that allows for the insertion or removal of one or more cross-flow filters from the housing.

Some systems include two or more TFF units arranged in series or in parallel. For example, in systems where two or more TFF units are arranged in series, a fluid conduit can be used to fluidly connect two neighboring TFF units (e.g., any of the exemplary TFF units described herein or known in the art). One such exemplary arrangement of two TFF units in a system is shown in FIG. 4. The two or more TFF units arranged in series can be designed in any manner as long as the actuation of the at least one pump in the system results in the reversible flow of the cell culture from the reservoir, e.g., the bioreactor, through the first conduit, the two or more TFF units, one or more conduits positioned between the neighboring TFF unit(s), the second conduit, and back to the reservoir, e.g., the bioreactor). The two or more TFF units can be identical (e.g., same number and type of cross-flow filters) or different (e.g., different number and type of cross-flow filters). In some examples, the two or more TFF units each contain a single tubular cross-flow filter. Each TFF unit can be fluidly connected to a filtrate conduit that allows the filtrate leaving the TFF unit to be flowed into a filtrate holding tank (e.g., any of the filtrate holding tanks described herein). In some embodiments, the two or more TFF units can be disposed in a single housing (e.g., any of the exemplary types of housing described herein or known in the art).

### Pumps

The systems described herein can include one or more pumps. In some examples, the one or more pumps are low turbulence pumps (LTPs). LTPs care pumps that can move a fluid (e.g., cell culture) in a single direction (e.g., a first or second flow direction) or reversibly move a fluid (e.g., a cell culture) in two directions (a first and second flow direction) without inducing a substantial amount of sheer stress and/or fluid turbulence in the fluid (e.g., cell culture). When a LTP is used to flow a fluid (e.g., a cell culture) in alternating first and second flow directions, the second flow direction is approximately opposite to that of the first flow direction.

An example of an LTP pump is a peristaltic pump. A peristaltic pump can have pump head with a volume of between about between about 20 mL to about 250 mL (e.g., between about 20 mL and about 240 mL, between about 20 mL and about 220 mL, between about 20 mL and about 200 mL, between about 20 mL and about 180 mL, between about 20 mL and about 160 mL, between about 20 mL and about 140 mL, between about 20 mL and about 120 mL, between about 20 mL and about 100 mL, between about 20 mL and about 80 mL, between about 20 mL and about 60 mL, between about 20 mL and about 50 mL, between about 20 mL and about 40 mL, between about 20 mL and about 30 mL, between about 30 mL and about 240 mL, between about 30 mL and about 220 mL, between about 30 mL and about 200 mL, between about 30 mL and about 180 mL, between about 30 mL and about 160 mL, between about 30 mL and about 140 mL, between about 30 mL and about 120 mL, between about 30 mL and about 100 mL, between about 30 mL and about 80 mL, between about 30 mL and about 60 mL, between about 40 mL and about 250 mL, between about 40 mL and about 240 mL, between about 40 mL and about 220 mL, between about 40 mL and about 200 mL, between about 40 mL and about 180 mL, between about 40 mL and about 160 mL, between about 40 mL and about 140 mL, between about 40 mL and about 120 mL, between about 40 mL and about 100 mL, between about 40 mL and about 80 mL, between about 40 mL and about 60 mL, between about 50 mL and about 250 mL, between about 50 mL and about 240 mL, between about 50 mL and about 220 mL, between about 50 mL and about 200 mL, between about 50 mL and about 180 mL, between about 50 mL and about 160 mL, between about 50 mL and about 140 mL, between about 50 mL and about 120 mL, between about 50 mL and about 100 mL, between about 50 mL and about 80 mL, between about 50 mL and about 75 mL, between about 60 mL and about 250 mL, between about 60 mL and about 240 mL, between about 60 mL and about 220 mL, between about 60 mL and about 200 mL, between about 60 mL and about 180 mL, between about 60 mL and about 160 mL, between about 60 mL and about 140 mL, between about 60 mL and about 120 mL, between about 60 mL and about 100 mL, between about 60 mL and about 80 mL, between about 70 mL and about 250 mL, between about 70 mL and about 240 mL, between about 70 mL and about 220 mL, between about 70 mL and about 200 mL, between about 70 mL and about 180 mL, between about 70 mL and about 160 mL, between about 70 mL and about 140 mL, between about 70 mL and about 120 mL, between about 70 mL and about 100 mL, between about 80 mL and about 250 mL, between about 80 mL and about 240 mL, between about 80 mL and about 220 mL, between about 80 mL and about 200 mL, between about 80 mL and about 180 mL, between about 80 mL and about 160 mL, between about 80 mL and about 140 mL, between about 80 mL and about 120 mL, between about 80 mL and about 100 mL, between about 90 mL and about 250 mL, between about 90 mL and about 240 mL, between about 90 mL and about 220 mL, between about 90 mL and about 200 mL, between about 90 mL and about 180 mL, between about 90 mL and about 160 mL, between about 90 mL and about 140 mL, between about 90 mL and about 120 mL, between about 90 mL and about 100 mL, between about 100 mL and about 250 mL, between about 100 mL and about 240 mL, between about 100 mL and about 220 mL, between about 100 mL and about 200 mL, between about 100 mL and about 180 mL, between about 100 mL and about 160 mL, between about 100 mL and about 140 mL, or between about 100 mL and about 120 mL). The peristaltic pump can have tubing with an internal diameter of between about 5 mm and about 400 mm (e.g., between about 5 mm and about 380 mm, between about 5 mm and about 360 mm, between about 5 mm and about 340 mm, between about 5 mm and about 320 mm, between about 5 mm and about 300 mm, between about 5 mm and about 280 mm, between about 5 mm and about 260 mm, between about 5 mm and about 240 mm, between about 5 mm and about 220 mm, between about 5 mm and about 200 mm, between about 5 mm and about 180 mm, between about 5 mm and about 160 mm, between about 5 mm and about 140 mm, between about 5 mm and about 120 mm, between about 5 mm and about 100 mm, between about 5 mm and about 80 mm, between about 5 mm and about 60 mm, between about 5 mm and about 55 mm, between about 5 mm and about 50 mm, between about 5 mm and about 45 mm, between about 5 mm and about 40 mm, between about 5 mm and about 35 mm, between about 5 mm and about 30 mm, between about 5 mm and about 25 mm, between about 5 mm and about 20 mm, between about 5 mm and about 15 mm, between about 5 mm and about 10 mm, between about 1 mm and about 10 mm, between about 10 mm and about 60 mm, between about 10 mm and about 35 mm, between about 10 mm and about 25 mm, between about 10 mm and about 20 mm, between about 20 mm and about 60 mm, between about 20 mm and about 50 mm, or between about 30 mm and about 50 mm). The tubing within a peristaltic pump can have a wall diameter of between about 1 mm to about 30 mm (e.g., between about 1 mm to about 25 mm, between about 1 mm to about 20 mm, between about 1 mm to about 18 mm, between about 1 mm to about 16 mm, between about 1 mm to about 14 mm, between about 1 mm to about 12 mm, between about 1 mm to about 10 mm, between about 1mm to about 8 mm, between about 1 mm to about 6 mm, or between about 1 mm to about 5 mm). Examples of peristaltic pump(s) that can be used in the present systems and methods are Watson Marlow 620 and Watson Marlow 800 pumps. Any of the peristaltic pumps described herein can have a twin channel and/or contain GORE Sta-Pure tubing (e.g., tubing with an internal diameter of 16 mm and a 4 mm wall).

Additional examples of LTP pumps are described in U.S. Patent Nos. 4,037,984; 5,033,943; and 5,458,459; U.S. Patent Application Publication No. 2009/0199904, and international patent application number WO 06/021873. Other examples of LTP pumps include rotary positive displacement pumps, lobe pumps, internal gear pumps, and progressive cavity pumps. Those skilled in the art will appreciate that other types of LTPs are commercially available and can be used in any of the systems and methods described herein.

In some examples, the at least one pump is disposed in the first or the second conduit, or both. In other examples, the at least one pump is disposed in the reservoir and proximal to the first or second fluid conduit. In systems that include two or more TFF units, at least one pump can be disposed in a conduit placed between two neighboring TFF units (e.g., conduit **14** shown in FIG. 4). The at least one pump can be disposed anywhere in the systems provided herein as long as upon actuation of the at least one pump results fluid flowing reversibly through the system from the reservoir, through the first conduit, the TFF unit, the second conduit, and back to the reservoir, or in systems containing two or more TFF units, fluid flowing reversibly through the system from the reservoir, through the first conduit, the two or more TFF units, the one or more conduits between neighboring TFF units, the second conduit, and back to the reservoir.

*Filtrate Holding Tank*

A filtrate holding tank can optionally be included in the system, e.g., to store the filtrate. For example, the filtrate can be stored for a period of between about 1 hour and about one week (e.g., between about 1 hour and about 6 days, between about 1 hour and about 5 days, between about 1 hour and about 4 days, between about 1 hour and about 3 days, between about 1 hour and about 2 days, between about 1 hour and about 36 hours, between about 1 hour and about 24 hours, between about 1 hour and about 20 hours, between about 1 hour and about 16 hours, between about 1 hour and about 12 hours, or between about 1 hour and about 6 hours). The filtrate holding tank can have an internal volume of between about 50 mL and about 50 L (e.g., between about 50 mL and about 45 L, between about 50 mL and about 40 L, between about 50 mL and about 35 L, between about 50 mL and about 30 L, between about 50 mL and about 25 L, between about 50 mL and about 20 L, between about 50 mL and about 18 L, between about 50 mL and about 16 L, between about 50 mL and about 14 L, between about 50 mL and about 12 L, between about 50 mL and about 10 L, between about 50 mL and about 9 L, between about 50 mL and about 8 L, between about 50 mL and about 7 L, between about 50 mL and about 6 L, between about 50 mL and about 5 L, between about 50 mL and about 4.5 L, between about 50 mL and about 4.0 L, between about 50 mL and about 3.5 L, between about 50 mL and about 3.0 L, between about 50 mL and about 2.5 L, between about 50 mL and about 2.0 L, between about 50 mL and about 1.5 L, between about 50 mL and about 1.0 L, between about 100 mL and about 1.0 L, or between about 500 mL and about 1.0 L). The interior surface of the filtrate holding tank can contain a biocompatible material (e.g., any biocompatible material known in the art). The filtrate holding tank can be a refrigerated holding tank that is capable of storing the filtrate at a temperature of between about 10 ° C and about 35 ° C (e.g., between about 10 °C and about 30 °C, between about 10 °C and about 25 °C, between about 10 °C and about 20 °C, between about 10 °C and about 15° C, or between about 15 °C and about 25 °C). As one of skill in the art can appreciate, a number of different commercially available holding tanks can be used as a filtrate holding tank in the systems and methods described herein.

*Flowmeters*

Some examples of the systems described herein can include one or more (e.g., two, three, four, or five) flowmeters. For example, the one or more flowmeters can be disposed in one or more of any of the conduits in the system (e.g., the first conduit, the second conduit, the one or more conduits between neighboring TFF units, and/or the filtrate conduit). For example, a flowmeter can be placed in between two neighboring TFF units. In some examples, the flowmeter(s) is/are non-invasive. Those skilled in the art would understand the wide variety of commercially-available flowmeters that can be used in the present systems and methods. For example, a EM-TEC BioProTT non-invasive, real-time flowmeter, a PT878 Ultrasonic Flowmeter (Rshydro), and a Sono-Trak ultrasonic non-invasive flowmeter (EMCO) are commercially available flowmeters that can be used in the present systems and methods.

*Pressure Sensors*

The systems described herein can include one or more pressure sensors. For example, the one or more pressure sensors can be disposed in any of the conduits in the system (e.g., the first conduit, the second conduit, the one or more conduits between neighboring TFF units, and/or the filtrate conduit). For example, a pressure sensor can be placed in between two neighboring TFF units in a system. Those skilled in the art would understand the wide variety of commercially-available pressure sensors that can be used in the present systems and methods. A non-limiting example of pressure sensor that can be used in the systems and methods described herein is a PendoTECH PressureMAT pressure sensor.

*Clamps/Ports*

Any of the systems described herein can optionally include a port conduit between the first or second conduit and a port that fluidly connects the first or second conduit, respectively, to the port. The port can be used to deliver or remove a fluid (e.g., cell culture or washing solution) from the system (through the first or second conduit, respectively). A clamp can be disposed in the port conduit. A wide variety of suitable clamps are known in the art (e.g., a screw clamp). The port conduit can have any combination of the features described above for conduits. The port can be any type of port commonly known in the art. For example, a port can be an injection port or can have a ribbed threading.

*Biological Manufacturing Systems*

Any of the devices described herein can include a biological manufacturing system that includes at least one (e.g., two, three, or four) multi-column chromatography system (MCCS) having an inlet and outlet, and a filtrate conduit between the TFF unit or the filtrate holding tank, where the device is configured such that the filtrate is passed into the inlet of the biological manufacturing system, through the at least one MCCS, and exits the device through the outlet of the biological manufacturing system. A MCCS can include two or more chromatography columns, two or more chromatographic membranes, or a combination of at least one chromatography column and at least one chromatographic membrane. In non-limiting examples, a MCCS can include four chromatographic columns, three chromatographic columns and a chromatographic membrane, three chromatographic columns, two chromatographic columns, two chromatographic membranes, and two chromatographic columns and one chromatographic membrane. Additional examples of combinations of chromatography columns and/or chromatographic membranes can be envisioned for use in an MCCS by one skilled in the art without limitation. The individual chromatography columns and/or chromatographic membranes present in a MCCS can be identical (e.g., have the same shape, volume, resin, capture mechanism, and unit operation), or can be different (e.g., have one or more of a different shape, volume, resin, capture mechanism, and unit operation). The individual chromatography column(s) and/or chromatographic membrane(s) present in a MCCS can perform the same unit operation (e.g., the unit operation of capturing, purifying, polishing, inactivating viruses, adjusting the ionic concentration and/or pH of a fluid containing the recombinant therapeutic protein, or filtering) or different unit operations (e.g., different unit operations selected from, e.g., the group of capturing, purifying, polishing, inactivating viruses, adjusting the ionic concentration and/or pH of a fluid containing the recombinant therapeutic protein, and filtering).

One or more (e.g., three, four, five, six, seven, eight, nine, ten, eleven, twelve, thirteen, fourteen, fifteen, sixteen, seventeen, eighteen, nineteen, twenty, twenty-one, twenty-two, twenty-three, or twenty-four) different types of buffer can be employed during the use of the one or more MCCS(s) in any of the biological manufacturing devices described herein. As is known in the art, the one or more types of buffer used in the one or more MCCSs used in the biological manufacturing systems described herein will depend on the resin present in the chromatography column(s) and/or the chromatographic membrane(s) of the one or more MCCSs (e.g., the first and second MCCSs), the recombinant therapeutic protein, and unit operation (e.g., any of the exemplary unit operations described herein) performed by the specific chromatography column(s) and/or chromatography membranes of the one or more MCCSs. The volume and type of buffer employed during the use of the one or more MCCSs in any of the biological processing devices described herein can also be determined by one skilled in the art. For example, the volume and type(s) of buffer employed during the use of the one or more MCCSs in any of the processes described herein can be chosen in order to optimize one or more of the following in the resulting isolated recombinant protein (e.g., drug product): the overall yield of recombinant therapeutic protein, the activity of the recombinant therapeutic protein, the level of purity of the recombinant therapeutic protein, and the removal of biological contaminants from a fluid containing the recombinant therapeutic protein (e.g., absence of active viruses, mycobacteria, yeast, bacteria, or mammalian cells).

The one or more MCCS can be a periodic counter current chromatography system (PCCS). A PCCS can, e.g., include two or more chromatography columns (e.g., three columns or four columns) that are switched in order to allow for the continuous elution of recombinant therapeutic protein from the two or more chromatography columns. A PCCS can include two or more chromatography columns, two or more chromatographic membranes, or at least one chromatographic column and at least one chromatographic membrane. A column operation generally consists of the load, wash, eluate, and regeneration steps. In PCCSs, multiple columns are used to run the same steps discretely and continuously in a cyclic fashion. Since the columns are operated in series, the flow through and wash from one column is captured by another column. This unique feature of PCCSs allows for loading of the resin close to its static binding capacity instead of to the dynamic binding capacity, as is typical during batch mode chromatography. As a result of the continuous cycling and elution, fluid entering a PCCS is processed continuously, and the eluate containing recombinant therapeutic protein is continuously produced.

The one or more unit operations that can be performed by the at least one MCCS in the biological manufacturing systems include, for example, capturing the recombinant therapeutic protein, inactivating viruses present in a fluid containing the recombinant therapeutic protein, purifying the recombinant therapeutic protein, polishing the recombinant therapeutic protein, holding a fluid containing the recombinant therapeutic protein (e.g., using a break tank), filtering or removing particulate material from a fluid containing the recombinant therapeutic protein, and adjusting the ionic concentration and/or pH of a fluid containing the recombinant therapeutic protein.

The unit operation of capturing can be performed using one or more MCCSs that include(s) at least one chromatography column and/or chromatography resin, e.g., that utilizes a capture mechanism. Non-limiting examples of capturing mechanisms include a protein A-binding capture mechanism, an antibody- or antibody fragment-binding capture mechanism, a substrate-binding capture mechanism, an aptamer-binding capture mechanism, a tag-binding capture mechanism (e.g., poly-His tag-based capture mechanism), and a cofactor-binding capture mechanism. Capturing can also be performed using a resin that can be used to perform cation exchange or anion exchange chromatography, or molecular sieve chromatography. Examples of resins that can be used to capture a recombinant therapeutic protein are known in the art.

The unit operation of inactivating viruses present in a fluid containing the recombinant therapeutic protein can be performed using one or more MCCSs that include(s), e.g., a chromatography column, a chromatography membrane, or a holding tank that is capable of incubating a fluid containing the recombinant therapeutic protein at a pH of between about 3.0 to 5.0 (e.g., between about 3.5 to about 4.5, between about 3.5 to about 4.25, between about 3.5 to about 4.0, between about 3.5 to about 3.8, or about 3.75) for a period of at least 30 minutes (e.g., a period of between about 30 minutes to 1.5 hours, a period of between about 30 minutes to 1.25 hours, a period of between about 0.75 hours to 1.25 hours, or a period of about 1 hour).

The unit operation of purifying a recombinant protein can be performed using one or more MCCSs that include(s), e.g., a chromatography column or chromatographic membrane that contains a resin, e.g., that utilizes a capture system. Non-limiting examples of capturing mechanisms include a protein A-binding capture mechanism, an antibody- or antibody fragment-binding capture mechanism, a substrate-binding capture mechanism, an aptamer-binding capture mechanism, a tag-binding capture mechanism (e.g., poly-His tag-based capture mechanism), and a cofactor-binding capture mechanism. Purifying can also be performed using a resin that can be used to perform cation exchange or anion exchange chromatography, or molecular sieve chromatography. Examples of resins that can be used to purify a recombinant therapeutic protein are known in the art.

The unit operation of polishing a recombinant protein can be performed using one or more MCCSs that include(s), e.g., a chromatography column or chromatographic membrane that contains a resin, e.g., that can be used to perform cation exchange, anion exchange, or molecular sieve chromatography. Examples of resins that can be used to polish a recombinant therapeutic protein are known in the art.

The unit operation of holding a fluid containing the recombinant therapeutic protein can be performed using an MCCS that includes at least one reservoir (e.g., a break tank) or a maximum of 1, 2, 3, 4, or 5 reservoir(s) (e.g., break tank(s)) in the one or more MCCS(s) in the biological manufacturing system. For example, the reservoir(s) (e.g., break tank(s)) that can be used to achieve this unit operation can each have a volume of between about 1 mL to about 1 L (e.g., between about 1 mL to about 800 mL, between about 1 mL to about 600 mL, between about 1 mL to about 500 mL, between about 1 mL to about 400 mL, between about 1 mL to about 350 mL, between about 1 mL to about 300 mL, between about 10 mL and about 250 mL, between about 10 mL and about 200 mL, between about 10 mL and about 150 mL, and between about 10 mL to about 100 mL). The reservoir(s) (e.g., break tank(s)) used in the biological manufacturing systems described herein can have a capacity that is, e.g., between 1 mL and about 300 mL, inclusive, e.g., between 1 mL and about 280 mL, about 260 mL, about 240 mL, about 220 mL, about 200 mL, about 180 mL, about 160 mL, about 140 mL, about 120 mL, about 100 mL, about 80 mL, about 60 mL, about 40 mL, about 20 mL, or about 10 mL, inclusive. The reservoir(s) (e.g., break tank(s)) in the biological manufacturing system can each hold the fluid containing the recombinant therapeutic protein for at least 10 minutes (e.g., at least 20 minutes, at least 30 minutes, at least 1 hour, at least 2 hours, at least 4 hours, or at least 6 hours). In other examples, the reservoir(s) (e.g., break tank(s)) in the biological manufacturing system only holds a therapeutic protein for a total time period of, e.g., between about 5 minutes and less than about 6 hours, inclusive, e.g., between about 5 minutes and about 5 hours, about 4 hours, about 3 hours, about 2 hours, about 1 hour, or about 30 minutes, inclusive. The reservoir(s) (e.g., break tank(s)) in the biological manufacturing system can be used to both hold and refrigerate (e.g., at a temperature of less than 25 °C, less than 15 °C, or less than 10 °C) the fluid containing the recombinant therapeutic protein. The reservoir can have any shape, including a circular cylinder, an oval cylinder, or an approximately rectangular sealed and nonpermeable bag.

The unit operations of filtering a fluid containing the recombinant therapeutic protein can be performed using an MCCS that includes, e.g., a filter, or a chromatography column or chromatographic membrane that contains a molecule sieve resin. As is known in the art, a wide variety of submicron filters (e.g., a filter with a pore size of less than 1 μm, less than 0.5 μm, less than 0.3 μm, about 0.2 μm, less than 0.2 μm, less than 100 nm, less than 80 nm, less than 60 nm, less than 40 nm, less than 20 nm, or less than 10 nm) are available in the art that are capable of removing any precipitated material and/or cells (e.g., precipitated, unfolded protein; precipitated, unwanted host cell proteins; precipitated lipids; bacteria; yeast cells; fungal cells; and/or mycobacteria). Filters having a pore size of about 0.2 μm or less than 0.2 μm are known to effectively remove bacteria from the fluid containing the recombinant therapeutic protein. As is known in the art, a chromatography column or a chromatographic membrane containing a molecular sieve resin can also be used in an MCCS to perform the unit operation of filtering a fluid containing a recombinant therapeutic protein.

The unit operations of adjusting the ionic concentration and/or pH of a fluid containing the recombinant therapeutic protein can be performed using a MCCS that includes and utilizes a buffer adjustment reservoir (e.g., an in-line buffer adjustment reservoir) that adds a new buffer solution into a fluid that contains the recombinant therapeutic protein (e.g., between columns within a single MCCS, or after the last column in a penultimate MCCS and before the fluid containing the recombinant therapeutic protein is fed into the first column of the next MCCS (e.g., the second MCCS). As can be appreciated in the art, the in-line buffer adjustment reservoir can be any size (e.g., greater than 100 mL) and can contain any buffered solution (e.g., a buffered solution that has one or more of: an increased or decreased pH as compared to the fluid containing the recombinant therapeutic protein, a an increased or decreased ionic (e.g., salt) concentration compared to the fluid containing the recombinant therapeutic protein, and/or an increased or decreased concentration of an agent that competes with the recombinant therapeutic protein for binding to resin present in at least one chromatographic column or at least one chromatographic membrane in an MCCS (e.g., the first or the second MCCS)).

A MCCS can perform two or more unit operations. For example, a MCCS can perform at least the following unit operations: capturing the recombinant therapeutic protein and inactivating viruses present in the fluid containing the recombinant therapeutic protein; capturing the recombinant therapeutic protein, inactivating viruses present in the fluid containing the recombinant therapeutic protein, and adjusting the ionic concentration and/or pH of a liquid containing the recombinant therapeutic protein; purifying the recombinant therapeutic protein and polishing the recombinant therapeutic protein; purifying the recombinant therapeutic protein, polishing the recombinant therapeutic protein, and filtering a fluid containing the recombinant therapeutic protein or removing precipitates and/or particulate matter from a fluid containing the recombinant therapeutic protein; and purifying the recombinant therapeutic protein, polishing the recombinant therapeutic protein, filtering a fluid containing the recombinant therapeutic protein or removing precipitates and/or particular matter from a fluid containing the recombinant therapeutic protein, and adjusting the ionic concentration and/or pH of a liquid containing the recombinant therapeutic protein.

### *Benefits Provided by the Present Systems*

The systems described herein provide for the continuous filtration of cell culture that has one or more (e.g., two, three, four, five, six, or seven) of the following benefits: decreased external volume of cell culture (outside of the reservoir), increased exchange fraction (within the first conduit, the TFF unit, and the second conduit), decreased external residence time of cell culture (outside the reservoir), decreased shear stress during cell culture filtration, improved cell viability in cell culture, elevated viable cell density in cell culture, and decreased filter fouling as compared to other unidirectional open circuit filtration systems (e.g., unidirectional TFF systems) or bidirectional closed circuit filtration systems (closed circuit ATF™ systems).

The exchange fraction and external residence time of a system described herein can be calculated using Equations 1 and 2 below.

$$\text{Exchange fraction} = \frac{\text{exchange volume}}{\text{external volume}} \qquad \text{(Equation 1)}$$

$$\text{External residence time} = \frac{\text{external volume}}{\text{exchange rate x exchange fraction}} \qquad \text{(Equation 2)}$$

For example, the present systems can have only a total external volume of cell culture that is between about 1% and about 7% (e.g., between about 1.0% and about 6.5%, between about 1% and about 6.0%, between about 1% and about 5.5%, or between about 1% and about 5.0%) of the total volume of cell culture in reservoir, the first conduit, the second conduit, and the TFF unit. The systems provided herein can also provide for a reduced residence time of the cell culture outside of the reservoir (reduced external residence time) of between about 1 second and about 60 seconds (e.g., between about 1 second and about 55 seconds, between about 1 second and about 50 seconds, between about 1 second and 45 seconds, between about 1 second and about 30 seconds, between about 1 second and about 25 seconds, between about 1 second and about 20 seconds, between about 1 second and about 15 seconds, between about 1 second and 13 seconds, between about 1 second and 10 seconds, between about 1 second and about 8 seconds, between about 1 second and about 5 seconds, or between about 10 seconds and 14 seconds). Table 1 below compares the external residence time of the exemplary system described in the Example and a closed system alternating tangential filtration system (ATF4).

Table 1. Comparison of the External Residence Time and External Fraction of

Exemplary System Provided Herein and Closed System ATF4

|  | External Volume | External Fraction | Residence Time |
|---|---|---|---|
| ATF4 | 0.756 L | 19% | **71 s** |
| TFF | 0.550 L | 78% | **12 s** |

The present systems can provide for an improved exchange fraction of greater than about 50% (e.g., greater than about 55%, greater than about 60%, greater than about 65%, greater than about 70%, greater than about 75%, greater than about 80%, or greater than about 85%). The systems described herein can provide for high viable cell densities in cell culture, e.g., a viable cell density of greater than about $30 \times 10^6$ cells/mL, greater than about $32 \times 10^6$ cells/mL, greater than about $34 \times 10^6$ cells/mL, greater than about $36 \times 10^6$ cells/mL, greater than about $38 \times 10^6$ cells/mL, greater than about $40 \times 10^6$ cells/mL, greater than about $42 \times 10^6$ cells/mL, greater than about $44 \times 10^6$ cells/mL, greater than about $46 \times 10^6$ cells/mL, greater than about $48 \times 10^6$ cells/mL, greater than about $50 \times 10^6$ cells/mL, greater than about $52 \times 10^6$ cells/mL, greater than about $54 \times 10^6$ cells/mL, greater than about $56 \times 10^6$ cells/mL, greater than about $58 \times 10^6$ cells/mL, or greater than about $60 \times 10^6$ cells/mL. The systems described herein can provide for a viable cell density of greater than about $65 \times 10^6$ cells/mL, greater than about $70 \times 10^6$ cells/mL, greater than about $75 \times 10^6$ cells/mL, greater than about $80 \times 10^6$ cells/mL, greater than about $85 \times 10^6$ cells/mL, greater than about $90 \times 10^6$ cells/mL, greater than about $95 \times 10^6$ cells/mL, greater than about $100 \times 10^6$ cells/mL, greater than about $105 \times 10^6$ cells/mL, greater than about $110 \times 10^6$ cells/mL, greater than about $115 \times 10^6$ cells/mL, greater than about $120 \times 10^6$ cells/mL, greater than about $125 \times 10^6$ cells/mL, greater than about $130 \times 10^6$ cells/mL, greater than about $135 \times 10^6$ cells/mL, greater than about $140 \times 10^6$ cells/mL, greater than about $145 \times 10^6$ cells/mL, greater than about $150 \times 10^6$ cells/mL, greater than about $155 \times 10^6$ cells/mL, greater than about $160 \times 10^6$ cells/mL, greater than about $165 \times 10^6$ cells/mL, greater than about $170 \times 10^6$ cells/mL, greater than about $175 \times 10^6$ cells/mL, greater than about $180 \times 10^6$ cells/mL, greater than about $185 \times 10^6$ cells/mL, greater than about $190 \times 10^6$ cells/mL, greater than about $200 \times 10^6$ cells/mL, greater than about $210 \times 10^6$ cells/mL, greater than about $220 \times 10^6$ cells/mL, greater than about $230 \times 10^6$ cells/mL, greater than about $240 \times 10^6$ cells/mL, or greater than about $250 \times 10^6$ cells/mL.

The systems provided herein also provide for an optimized exchange rate (also called flow rate herein). As can be appreciated by those in the art, an exchange rate that is too high can result in a level of shear stress that negatively impacts cell growth and cell culture performance, and an exchange rate that is too low can result in filter fouling and longer external residence time of the cell culture. The systems provided herein provide for the achievement of any of the exemplary flow rates described herein.

The systems provided herein also provide for an optimized exchange rate (XR) to perfusion rate (PR) ratio. As one of skill can appreciate, systems and methods that provide increased ratios of XR:PR result in more efficient cell culture production methods (e.g., utilize less cell culture medium during the perfusion process). In some examples, the exemplary devices and methods herein provide for a XR:PR ratio of greater than about 2 (e.g., greater than about 3, greater than about 4, greater than about 5, greater than about 6, greater than about 7, greater than about 8, greater than about 9, greater than about 10, greater than about 11, greater than about 12, greater than about 13, greater than about 14, greater than about 15, greater than about 16, greater than about 17, greater than about 18, greater than about 19, greater than about 20, greater than about 21, greater than about 22, greater than about 23, greater than about 24, greater than about 25, greater than about 50, greater than about 75, greater than about 100, greater than about 125, greater than about 150, greater than about 175, greater than about 200, greater than about 225, greater than about 250, greater than about 275, greater than about 300, greater than about 325, greater than about 350, greater than about 375, greater than about 400, greater than about 425, greater than about 450, greater than about 475, greater than about 500, greater than about 525, greater than about 550, greater than about 575, or greater than about 600), or between about 5 and about 600 (e.g., between about 10 and about 550, between about 10 and about 500, between about 10 and about 450, between about 10 and about 400, between about 10 and about 350, between about 10 and about 300, between about 10 and about 250, between about 10 and about 200, between about 10 and about 150, between about 10 and about 100, or between about 10 and about 50).

**Methods of Processing Cell Culture**

Also provided are methods of processing a cell culture that include (a) providing an open circuit filtration system (e.g., any of the open circuit filtration systems described herein), (b) flowing cell culture from the reservoir through the TFF unit in a first flow direction for a first period of time, (c) reversing the first flow direction and flowing the cell culture through the TFF unit in a second flow direction for a second period of time, (d) reversing the second flow direction and flowing the culture through the TFF unit in the first flow direction for a third period of time, (e) repeating steps (c)-(d) at least two (e.g., at least three, four, five, six, seven, eight, nine, ten, fifteen, twenty, thirty, forty, fifty, sixty, seventh, eighty, ninety, or one hundred, or more than one hundred) times, and (f) collecting the filtrate. Various exemplary aspects of these methods are described below.

*Cell Culture*

The cell culture to be processed in the methods provided herein can contain a plurality of any type of mammalian cell in a liquid culture medium. In some examples of all the methods described herein, the mammalian is a cell that grows in suspension culture. In other examples, the mammalian cell is an adherent cell (e.g., a cell that requires a solid substrate, such as microcarriers, for growth in a perfusion bioreactor). Non-limiting examples of mammalian cells that can be present in a cell culture include: Chinese hamster ovary (CHO) cells (e.g., CHO DG44 cells, CHO-Kls cells, Sp2.0, myeloma cells (e.g., NS/0), B-cells, hybridoma cells, T-cells, human embryonic kidney (HEK) cells (e.g, HEK 293E and HEK 293F), African green monkey kidney epithelial cells (Vero) cells, and Madin-Darby Canine (Cocker Spaniel) kidney epithelial cells (MDCK) cells. Additional mammalian cells that can be present in a cell culture are known in the art.

A cell culture processed using any of the methods described herein can contain a viable cell density of greater than about $0.5 \times 10^6$ cells/mL, greater than about $1.0 \times 10^6$ cells/mL, greater than about $5.0 \times 10^6$ cells/mL, greater than about $10.0 \times 10^6$ cells/mL, greater than about $15.0 \times 10^6$ cells/mL, greater than about $20.0 \times 10^6$ cells/mL, greater than about $25.0 \times 10^6$ cells/mL, greater than about $30.0 \times 10^6$ cells/mL, greater than about $35.0 \times 10^6$ cells/mL, greater than about $40.0 \times 10^6$ cells/mL, greater than about $45.0 \times 10^6$ cells/mL, greater than about $50.0 \times 10^6$ cells/mL, greater than about $55.0 \times 10^6$ cells/mL, greater than about $60.0 \times 10^6$ cells/mL, greater than about $65.0 \times 10^6$ cells/mL, greater than about $70.0 \times 10^6$ cells/mL, greater than about $75.0 \times 10^6$ cells/mL, greater than about $80.0 \times 10^6$ cells/mL, greater than about $85.0 \times 10^6$ cells/mL, greater than about $90.0 \times 10^6$ cells/mL, greater than about $95.0 \times 10^6$ cells/mL, greater than about $100.0 \times 10^6$ cells/mL, greater than about $105.0 \times 10^6$ cells/mL, greater than about $110.0 \times 10^6$ cells/mL, greater than about $120.0 \times 10^6$ cells/mL, greater than about $125.0 \times 10^6$ cells/mL, greater than about $130.0 \times 10^6$ cells/mL, greater than about $135.0 \times 10^6$ cells/mL, greater than about $140.0 \times 10^6$ cells/mL, greater than about $145.0 \times 10^6$ cells/mL, greater than about $150.0 \times 10^6$ cells/mL, greater than about $155.0 \times 10^6$ cells/mL, greater than about $160.0 \times 10^6$ cells/mL, greater than about $170.0 \times 10^6$ cells/mL, greater than about $175.0 \times 10^6$ cells/mL, greater than about $180.0 \times 10^6$ cells/mL, greater than about $185.0 \times 10^6$ cells/mL, greater than about $190.0 \times 10^6$ cells/mL, greater than about $195.0 \times 10^6$ cells/mL, greater than about $200.0 \times 10^6$ cells/mL, greater than about $205.0 \times 10^6$ cells/mL, greater than about $210.0 \times 10^6$ cells/mL, greater than about $215.0 \times 10^6$ cells/mL, greater than about $220.0 \times 10^6$ cells/mL, greater than about $225.0 \times 10^6$ cells/mL, greater than about $230.0 \times 10^6$ cells/mL, greater than about $235. 0 \times 10^6$ cells/mL, greater than about $240.0 \times 10^6$ cells/mL, greater than about $245.0 \times 10^6$ cells/mL, or greater than about $250.0 \times 10^6$ cells/mL). In some examples, the cell culture has a viable cell concentration of between about $30 \times 10^6$ cells/mL and about $100 \times 10^6$ cells/mL (e.g., between about $30 \times 10^6$ cells/mL and about $95 \times 10^6$ cells/mL, between about $30 \times 10^6$ cells/mL and about $90 \times 10^6$ cells/mL, between about $30 \times 10^6$ cells/mL and about $85 \times 10^6$ cells/mL, between about $35 \times 10^6$ cells/mL and about $80 \times 10^6$ cells/mL, between about $40 \times 10^6$ cells/mL and about $80 \times 10^6$ cells/mL, between about $40 \times 10^6$ cells/mL and about $60 \times 10^6$ cells/mL, or between about $60 \times 10^6$ cells/mL and about $80 \times 10^6$ cells/mL). In some examples, the cell culture has a viable cell concentration of between about $110 \times 10^6$ cells/mL and about $250 \times 10^6$ cells/mL (e.g., between about $110 \times 10^6$ cells/mL and about $240 \times 10^6$ cells/mL, between about $110 \times 10^6$ cells/mL and about $230 \times 10^6$ cells/mL, between about $110 \times 10^6$ cells/mL and about $220 \times 10^6$ cells/mL, between about $110 \times 10^6$ cells/mL and about $210 \times 10^6$ cells/mL, between about $110 \times 10^6$ cells/mL and about $200 \times 10^6$ cells/mL, between about $110 \times 10^6$ cells/mL and about $190 \times 10^6$ cells/mL, between about $110 \times 10^6$ cells/mL and about $180 \times 10^6$ cells/mL, between about $110 \times 10^6$ cells/mL and about $170 \times 10^6$ cells/mL, between about $110 \times 10^6$ cells/mL and about $160 \times 10^6$ cells/mL, between about $110 \times 10^6$ cells/mL and about $150 \times 10^6$ cells/mL, between about $110 \times 10^6$ cells/mL and about $140 \times 10^6$ cells/mL, between about $110 \times 10^6$ cells/mL and about $130 \times 10^6$ cells/mL, between about $120 \times 10^6$ cells/mL and about $250 \times 10^6$ cells/mL, between about $120 \times 10^6$ cells/mL and about $240 \times 10^6$ cells/mL, between about $120 \times 10^6$ cells/mL and about $230 \times 10^6$ cells/mL, between about $120 \times 10^6$ cells/mL and about $220 \times 10^6$ cells/mL, between about $120 \times 10^6$ cells/mL and about $210 \times 10^6$ cells/mL, between about $120 \times 10^6$ cells/mL and about $200 \times 10^6$ cells/mL, between about $120 \times 10^6$ cells/mL and about $190 \times 10^6$ cells/mL, between about $120 \times 10^6$ cells/mL and about $180 \times 10^6$ cells/mL, between about $120 \times 10^6$ cells/mL and about $170 \times 10^6$ cells/mL, between about $120 \times 10^6$ cells/mL and about $160 \times$

$10^6$ cells/mL, between about 120 x $10^6$ cells/mL and about 150 x $10^6$ cells/mL, between about 120 x $10^6$ cells/mL and about 140 x $10^6$ cells/mL, between about 130 x $10^6$ cells/mL and about 250 x $10^6$ cells/mL, between about 130 x $10^6$ cells/mL and about 240 x $10^6$ cells/mL, between about 130 x $10^6$ cells/mL and about 230 x $10^6$ cells/mL, between about 130 x $10^6$ cells/mL and about 220 x $10^6$ cells/mL, between about 130 x $10^6$ cells/mL and about 210 x $10^6$ cells/mL, between about 130 x $10^6$ cells/mL and about 200 x $10^6$ cells/mL, between about 130 x $10^6$ cells/mL and about 190 x $10^6$ cells/mL, between about 130 x $10^6$ cells/mL and about 180 x $10^6$ cells/mL, between about 130 x $10^6$ cells/mL and about 170 x $10^6$ cells/mL, between about 130 x $10^6$ cells/mL and about 160 x $10^6$ cells/mL, between about 130 x $10^6$ cells/mL and about 150 x $10^6$ cells/mL, between about 140 x $10^6$ cells/mL and about 250 x $10^6$ cells/mL, between about 140 x $10^6$ cells/mL and about 240 x $10^6$ cells/mL, between about 140 x $10^6$ cells/mL and about 230 x $10^6$ cells/mL, between about 140 x $10^6$ cells/mL and about 220 x $10^6$ cells/mL, between about 140 x $10^6$ cells/mL and about 210 x $10^6$ cells/mL, between about 140 x $10^6$ cells/mL and about 200 x $10^6$ cells/mL, between about 140 x $10^6$ cells/mL and about 190 x $10^6$ cells/mL, between about 140 x $10^6$ cells/mL and about 180 x $10^6$, between about 140 x $10^6$ cells/mL and about 170 x $10^6$ cells/mL, between about 140 x $10^6$ cells/mL and about 160 x $10^6$ cells/mL, between about 150 x $10^6$ cells/mL and about 250 x $10^6$ cells/mL, between about 150 x $10^6$ cells/mL and about 240 x $10^6$ cells/mL, between about 150 x $10^6$ cells/mL and about 230 x $10^6$ cells/mL, between about 150 x $10^6$ and about 220 x $10^6$ cells/mL, between about 150 x $10^6$ cells/mL and about 210 x $10^6$ cells/mL, between about 150 x $10^6$ cells/mL and about 200 x $10^6$ cells/mL, between about 150 x $10^6$ cells/mL and about 190 x $10^6$ cells/mL, between about 150 x $10^6$ cells/mL and about 180 x $10^6$ cells/mL, between about 150 x $10^6$ cells/mL and about 170 x $10^6$ cells/mL, between about 160 x $10^6$ cells/mL and about 250 x $10^6$ cells/mL, between about 160 x $10^6$ cells/mL and about 240 x $10^6$ cells/mL, between about 160 x $10^6$ cells/mL and about 230 x $10^6$ cells/mL, between about 160 x $10^6$ cells/mL and about 220 x $10^6$ cells/mL, between about 160 x $10^6$ cells/mL and about 210 x $10^6$ cells/mL, between about 160 x $10^6$ and about 200 x $10^6$ cells/mL, between about 160 x $10^6$ cells/mL and about 190 x $10^6$ cells/mL, between about 160 x $10^6$ cells/mL and about 180 x $10^6$ cells/mL, between about 170 x $10^6$ cells/mL and about 250 x $10^6$ cells/mL, between about 170 x $10^6$ cells/mL and about 240 x $10^6$ cells/mL, between about 170 x $10^6$ cells/mL and about 230 x $10^6$ cells/mL, between about 170 x $10^6$ cells/mL and about 220 x $10^6$ cells/mL, between about 170 x $10^6$ cells/mL and about 210 x $10^6$ cells/mL, between about 170 x $10^6$ cells/mL and about 200 x $10^6$ cells/mL, between about 170 x $10^6$ cells/mL and about 190 x $10^6$ cells/mL, between about 180 x $10^6$ cells/mL and about 250 x $10^6$ cells/mL, between about 180 x $10^6$ cells/mL and about 240 x $10^6$ cells/mL, between about 180 x $10^6$ cells/mL and about 230 x $10^6$ cells/mL, between about 180 x $10^6$ cells/mL and about 220 x $10^6$ cells/mL, between about 180 x $10^6$ cells/mL and about 210 x $10^6$ cells/mL, between about 180 x $10^6$ cells/mL and about 200 x $10^6$ cells/mL, between about 190 x $10^6$ cells/mL and about 250 x $10^6$ cells/mL, between about 190 x $10^6$ cells/mL and about 240 x $10^6$ cells/mL, between about 190 x $10^6$ cells/mL and about 230 x $10^6$ cells/mL, between about 190 x $10^6$ cells/mL and about 220 x $10^6$ cells/mL, between about 190 x $10^6$ cells/mL and about 210 x $10^6$ cells/mL, between about 200 x $10^6$ cells/mL and about 250 x $10^6$ cells/mL, between about 200 x $10^6$ cells/mL and about 240 x $10^6$ cells/mL, between about 200 x $10^6$ cells/mL and about 230 x $10^6$ cells/mL, between about 200 x $10^6$ cells/mL and about 220 x $10^6$ cells/mL, between about 210 x $10^6$ cells/mL and about 250 x $10^6$ cells/mL, between about 210 x $10^6$ cells/mL and about 240 x $10^6$ cells/mL, between about 220 x $10^6$ cells/mL and about 240 x $10^6$ cells/mL, or between about 230 x $10^6$ cells/mL and about 250 x $10^6$ cells/mL).

The total amount of cell culture in the system (with the exception of the filtrate conduit and the filtrate holding tank) can be between 0.2 L and about 10,000 L (e.g., between about 0.2 L and about 9,500 L, between about 0.2 L and about 9,000 L, between about 0.2 L and about 8,500 L, between about 0.2 L and about 8,000 L, between about 0.2 L and about 7,500 L, between about 0.2 L and about 7,000 L, between about 0.2 L and about 6,500 L, between about 0.2 L and about 6,500 L, between about 0.2 L and about 6,000 L, between about 0.2 L and about 5,500 L, between about 0.2 L and about 5,000 L, between about 0.2 L and about 4,500 L, between about 0.2 L and about 4,000 L, between about 0.2 L and about 3,500 L, between about 0.2 L and about 3,000 L, between about 0.2 L and about 2,500 L, between about 0.2 L and about 2,000 L, between about 0.2 L and about 1,500 L, between about 0.2 L and about 1,000 L, between about 0.2 L and about 500 L, between about 0.2 L and about 400L, between about 0.2 L and about 300 L, between about 0.2 L and about 200 L, between about 0.2 L and about 150 L, between about 0.2 L and about 100 L, between about 0.2 L and about 50 L, or between about 0.2 L and about 10 L).

The mammalian cells present in a cell culture can contain a recombinant nucleic acid (e.g., a nucleic acid stably integrated in the mammalian cell's genome) that encodes a recombinant protein (e.g., a recombinant protein that is secreted by the mammalian cell). A nucleic acid encoding a recombinant protein can be introduced into a mammalian cell using a wide variety of methods known in molecular biology and molecular genetics. Non-limiting examples include transfection (e.g., lipofection), transduction (e.g., lentivirus, adenovirus, or retrovirus infection), and electroporation. In some instances, the nucleic acid that encodes a recombinant protein is not stably integrated into a chromosome of the mammalian cell (transient transfection), while in others the nucleic acid is integrated. Alternatively or in addition, the nucleic acid encoding a recombinant protein can be present in a plasmid and/or in a mammalian artificial chromosome (e.g., a human artificial chromosome). Alternatively or in addition, the nucleic acid can be introduced into the cell using a viral vector (e.g., a lentivirus, retrovirus, or adenovirus vector). The nucleic acid can be operably linked to a promoter sequence (e.g., a strong promoter, such as a β-actin promoter and CMV promoter, or an inducible promoter). A nucleic acid sequence encoding a soluble recombinant protein can contain a sequence that encodes a secretion signal peptide at the N- or C-terminus of the recombinant protein, which is cleaved by an enzyme present in the mammalian cell, and subsequently released into the culture medium. A vector containing the nucleic acid can, if desired, also contain a selectable marker (e.g., a gene that confers hygromycin, puromycin, or neomycin resistance to the mammalian cell).

Non-limiting examples of recombinant proteins that can be secreted by the mammalian cells in the cell culture include immunoglobulins (including light and heavy chain immunoglobulins, antibodies, or antibody fragments (e.g., any of the antibody fragments described herein), enzymes (e.g., a galactosidase (e.g., an alpha-galactosidase), Myozyme, or Cerezyme), proteins (e.g., human erythropoietin, tumor necrosis factor (TNF), or an interferon alpha or beta), or immunogenic or antigenic proteins or protein fragments (e.g., proteins for use in a vaccine). In some embodiments, the recombinant protein is an engineered antigen-binding polypeptide that contains at least one multifunctional recombinant protein scaffold (see, e.g., the recombinant antigen-binding proteins described in Gebauer et al., Current Opin. Chem. Biol. 13:245-255, 2009; and U.S. Patent Application Publication No. 2012/0164066). Non-limiting examples of recombinant proteins that are antibodies include: panitumumab, omalizumab, abagovomab, abciximab, actoxumab, adalimumab, adecatumumab, afelimomab, afutuzumab, alacizumab, alacizumab, alemtuzumab, alirocumab, altumomab, amatuximab, anatumomab, apolizumab, atinumab, tocilizumab, basilizimab, bectumomab, belimumab, bevacizumab, biciromab, canakinumab, cetuximab, daclizumab, densumab, eculizumab, edrecolomab, efalizumab, efungumab, ertumaxomab, etaracizumab, golimumab, infliximab, natalizumab, palivizumab, panitumumab, pertuzumab, ranibizumab, rituximab, tocilizumab, and trastuzumab. Additional examples of therapeutic antibodies that can be produced by the methods described herein are known in the art. Additional non-limiting examples of recombinant proteins that can be secreted by the mammalian cells in the cell culture include: alglucosidase alfa, laronidase, abatacept, galsulfase, lutropin alfa, antihemophilic factor, agalsidase beta, interferon beta-la, darbepoetin alfa, tenecteplase, etanercept, coagulation factor IX, follicle stimulating hormone, interferon beta-la, imiglucerase, dornase alfa, epoetin alfa, and alteplase.

Liquid culture media are known in the art. The liquid culture medium can be supplemented with a mammalian serum (e.g., fetal calf serum and bovine serum), and/or a growth hormone or growth factor (e.g., insulin, transferrin, and epidermal growth factor). Alternatively or in addition, the liquid culture medium can be a chemically-defined liquid culture medium, an animal-derived component free liquid culture medium, a serum-free liquid culture medium, or a serum-containing liquid culture medium. Examples of chemically-defined liquid culture media, animal-derived component free liquid culture media, serum-free liquid culture media, and serum-containing liquid culture media are commercially available.

A liquid culture medium typically contains an energy source (e.g., a carbohydrate, such as glucose), essential amino acids (e.g., the basic set of twenty amino acids plus cysteine), vitamins and/or other organic compounds required at low concentrations, free fatty acids, and/or trace elements. The liquid culture medium can, if desired, be supplemented with, e.g., a mammalian hormone or growth factor (e.g., insulin, transferrin, or epidermal growth factor), salts and buffers (e.g., calcium, magnesium, and phosphate salts), nucleosides and bases (e.g., adenosine, thymidine, and hypoxanthine), protein and tissue hydrolysates, and/or any combination of these or other additives.

Non-limiting examples of liquid culture media include, e.g., CD CHO, Opti CHO, and Forti CHO (all available from Life Technologies; Grand Island, NY), Hycell CHO medium (Thermo Fisher Scientific, Inc.; Waltham, MA), Ex-cell CD CHO Fusion medium (Sigma-Aldrich Co.; St. Louis, MO), and PowerCHO medium (Lonza Group, Ltd.; Basel, Switzerland). Medium components that also may be present in a liquid culture medium include, but are not limited to, chemically-defined (CD) hydrolysates, e.g., CD peptone, CD polypeptides (two or more amino acids), and CD growth factors. Additional examples of liquid tissue culture medium and medium components are known in the art.

A cell culture containing adherent mammalian cells can be grown in a perfusion bioreactor using, e.g., microcarriers. Non-limiting exemplary microcarriers that can be used include CytoPore™ 1 and CytoPore™ 2 (available from GE Healthcare, Life Sciences, Piscataway, New Jersey). Additional examples of microcarriers that can be used are publicly available and known in the art.

### Use of Exemplary Open Circuit Filtration Systems

Any of the open circuit filtration systems described herein can be used in the provided methods of processing a cell culture. For example, the bioreactor in the open circuit filtration system used in the methods described herein can be a bioreactor (e.g., any perfusion bioreactor known in the art) or a refrigerated holding tank. The open circuit filtration system used in the methods can include one or more conduits (e.g., the first conduit, the second conduit, the one or more conduits between neighboring TFF units, and/or the filtrate conduit) that is/are biocompatible tubing. In some examples, the open circuit filtration system contains a reservoir and two or more subsystems (as described herein).

The open circuit filtration systems used in the methods can include a TFF unit with a single cross-flow filter (e.g., a tubular cross-flow filter) or two or more (e.g., two, three, four, or five) cross-flow filters (e.g., tubular cross-flow filters) as described herein. In other examples, the open circuit filtration systems used can include two or more (e.g.,

two, three, or four) TFF units, where each pair of neighboring TFF units are fluidly connected by a fluid conduit. The TFF units can provide a total filtration area of between about 0.1 m$^2$ to about 150 m$^2$ (e.g., between about 0.1 m$^2$ to about 145 m$^2$, between about 0.1 m$^2$ and 140 m$^2$, between about 0.1 m$^2$ and about 135 m$^2$, between about 0.1 m$^2$ and about 130 m$^2$, between about 0.1 m$^2$ and about 125 m$^2$, between about 0.1 m$^2$ and about 120 m$^2$, between about 0.1 m$^2$ and about 115 m$^2$, between about 0.1 m$^2$ and about 110 m$^2$, between about 0.1 m$^2$ and about 105 m$^2$, between about 0.1 m$^2$ and about 100 m$^2$, between about 0.1 m$^2$ and about 95 m$^2$, between about 0.1 m$^2$ and about 90 m$^2$, between about 0.1 m$^2$ and about 85 m$^2$, between about 0.1 m$^2$ and 80 m$^2$, between about 0.1 m$^2$ and 75 m$^2$, between about 0.1 m$^2$ and about 70 m$^2$, between about 0.1 m$^2$ and about 65 m$^2$, between about 0.1 m$^2$ and 60 m$^2$, between about 0.1 m$^2$ and about 55 m$^2$, between about 0.1 m$^2$ and about 50 m$^2$, between about 0.1 m$^2$ and about 45 m$^2$, between about 0.1 m$^2$ and about 40 m$^2$, between about 0.1 m$^2$ and about 35 m$^2$, between about 0.1 m$^2$ and about 30 m$^2$, between about 0.1 m$^2$ and about 25 m$^2$, between about 0.1 m$^2$ and about 20 m$^2$, between about 0.1 m$^2$ and about 15 m$^2$, between about 0.1 m$^2$ and about 10 m$^2$, or between about 0.1 m$^2$ and about 5 m$^2$). The filter(s) present in a TFF unit can have any combination of the pore sizes (e.g., about 0.2 $\mu$m), shapes, fiber internal diameters, and/or fiber lengths described herein.

The open circuit filtration systems used herein can include at least one pump disposed in the first conduit or the second conduit, or both. The at least one pump can also be disposed in one or more of the conduits in the system (e.g., one or more of the first conduit, the second conduit, and/or the one or more conduits between neighboring TFF units). The system used can include at least one pump dislosed in the reservoir and proximal to the first or second conduit (e.g., a distance of between 0.01 cm to 5 cm (e.g., between 0.01 cm and 4 cm, between 0.01 cm and 3 cm, between 0.01 cm and 2 cm, or between 0.01 cm and 1 cm) from the pump to position where the first conduit or second conduit connects with the bioreactor). Some systems only include a single pump that flows the cell culture in the first direction during the first and third time periods, and flows the cell culture in the second direction during the second time period. Other systems include a first and a second pump, where the first pump flows the cell culture in the first direction and the second pump flows the cell culture in the second direction.

In any of the systems used in the methods, the at least one pump (e.g., one, two, three, or four pumps) can be a LTP (e.g., any of the LTPs described herein, such as a peristaltic pump). The at least one pump (e.g., at least one LTP) present in the system used in the methods can have any combination of the features or characteristics of pump (e.g., LTPs) described herein (e.g., pump head volume, type, and/or tubing). In some of the methods, the at least one pump is used at a pump speed (RPM) of between about 10 RPM and about 100 RPM (e.g., between about 10 RPM and about 95 RPM, between about 10 RPM and about 90 RPM, between about 10 RPM and about 85 RPM, between about 10 RPM and about 80 RPM, between about 10 RPM and about 75 RPM, between about 10 RPM and about 70 RPM, between about 10 RPM and about 65 RPM, between about 10 RPM and about 60 RPM, between about 10 RPM and about 55 RPM, between about 10 RPM and about 50 RPM, between about 10 RPM and about 45 RPM, between about 10 RPM and about 40 RPM, between about 10 RPM and about 35 RPM, between about 10 RPM and about 30 RPM, between about 10 RPM and about 25 RPM, or between about 10 RPM and about 20 RPM). In some examples, the methods result in a perfusion flux rate of between about 0.5 L/m$^2$/hour to about 40 L/m$^2$/hour, between about 0.5 L/m$^2$/hour to about 35 L/m$^2$/hour, between about 0.5 L/m$^2$/hour to about 30 L/m$^2$/hour, between about 0.5 L/m$^2$/hour and about 25 L/m$^2$/hour, between about 0.5 L/m$^2$/hour to about 20 L/m$^2$/hour, between about 0.5 L/m$^2$/hour to about 15 L/m$^2$/hour, between about 0.5 L/m2/hour to about 10 L/m$^2$/hour, between about 0.5 L/m$^2$/hour to about 9 L/m$^2$/hour, between about 0.5 L/m$^2$/hour to about 8 L/m$^2$/hour, between about 0.5 L/m$^2$/hour to about 7 L/m$^2$/hour, between about 0.5 L/m$^2$/hour to about 6 L/m$^2$/hour, between about 0.5 L/m$^2$/hour to about 5 L/m$^2$/hour, between about 0.5 L/m$^2$/hour to about 4 L/m$^2$/hour, between about 0.5 L/m$^2$/hour to about 3 L/m$^2$/hour, between about 0.5 L/m$^2$/hour to about 2 L/m$^2$/hour, or between about 0.8 L/m$^2$/hour to about 1.2 L/m$^2$/hour). In some examples, the use of the at least one pump results in a sheer rate in the system of between about 50 s$^{-1}$ to about 1000 s$^{-1}$ (e.g., between about 50 s$^{-1}$ to about 950 s$^{-1}$, between about 50 s$^{-1}$ to about 900 s$^{-1}$, between about 50 s$^{-1}$ to about 850 s$^{-1}$, between about 50 s$^{-1}$ to about 800 s$^{-1}$, between about 50 s$^{-1}$ to about 750 s$^{-1}$, between about 50 s$^{-1}$ to about 700 s$^{-1}$, between about 50 s$^{-1}$ to about 650 s$^{-1}$, between about 50 s$^{-1}$ to about 600 s$^{-1}$, between about 50 s$^{-1}$ to about 550 s$^{-1}$, between about 50 s$^{-1}$ to about 500 s$^{-1}$, between about s$^{-1}$ to about 450 s$^{-1}$, between about 50 s$^{-1}$ to about 400 s$^{-1}$, between about 50 s$^{-1}$ to about 350 s$^{-1}$, between about 50 s$^{-1}$ to about 300 s$^{-1}$, between about 50 s$^{-1}$ to about 250 s$^{-1}$, between about 50 s$^{-1}$ to about 200 s$^{-1}$, between about 50 s$^{-1}$ to about 150 s$^{-1}$, or between about 50 s$^{-1}$ to about 100 s$^{-1}$). Specific examples of pumps that can be used in these methods are a Watson-Marlow 620 peristaltic pump with 16 mm tubing or a Watson-Marlow 800 peristaltic pump with 40 mm tubing.

As one of skill in the art can appreciate, the total volume of cell culture in the system (excluding the volume of filtrate in the filtrate conduit and the filtrate holding tank), the total filtration area provided by the at least one TFF unit, and the flow rate (e.g., in the second and third time periods) needs to be performed at a reasonable ratio (e.g., exemplary values and parameters described herein) that allows for the one or more benefits of the presently provided systems and methods.

*Flow Cycle*

In the methods described herein, the first, second, and/or third periods of time can be between about 20 seconds and about 15 minutes (e.g., between about 30 seconds and about 15 minutes, between about 20 seconds and about 14 minutes, between about 20 seconds and about 13 minutes, between about 20 seconds and about 12 minutes, between about 20 seconds and about 11 minutes, between about 20 seconds and about 10 minutes, between about 20 seconds and about 9 minutes, between about 20 seconds and about 8 minutes, between about 20 seconds and about 7 minutes, between about 20 seconds and about 6 minutes, between about 20 seconds and about 5 minutes, between about 20 seconds and about 4 minutes, between about 20 seconds and about 3 minutes, between about 20 seconds and about 2 minutes, between about 20 seconds and about 115 seconds, between about 20 seconds and about 110 seconds, between about 20 seconds and 105 seconds, between about 20 seconds and about 100 seconds, between about 20 seconds and about 95 seconds, between about 20 seconds and about 90 seconds, between about 20 seconds and about 85 seconds, between about 20 seconds and about 80 seconds, between about 20 seconds and about 75 seconds, between about 20 seconds and about 70 seconds, between about 20 seconds and about 65 seconds, between about 20 seconds and about 60 seconds, between about 20 seconds and about 55 seconds, between about 20 seconds and about 50 seconds, between about 20 seconds and about 45 seconds, between about 20 seconds and about 40 seconds, between about 20 seconds and about 35 seconds, between about 20 seconds and about 30 seconds, between about 20 seconds and about 25 seconds, between about 30 seconds and about 90 seconds, between about 35 seconds and about 85 seconds, between about 40 seconds and about 80 seconds, between about 45 seconds and about 75 seconds, between about 50 seconds and about 70 seconds, between about 55 seconds and about 65 seconds, between about 30 seconds and 14 minutes, between about 30 seconds and 13 minutes, between about 30 seconds and 12 minutes, between about 30 seconds and about 11 minutes, between about 30 seconds and about 10 minutes, between about 30 seconds and about 9 minutes, between about 30 seconds and about 8 minutes, between about 30 seconds and about 7 minutes, between about 30 seconds and about 6 minutes, between about 30 seconds and about 5 minutes, between about 30 seconds and about 4 minutes, between about 30 seconds and about 3 minutes, between about 30 seconds and about 2 minutes, between about 30 seconds and about 90 seconds, between about 30 seconds and about 1 minute, between about 1 minute and about 15 minutes, between about 1 minute and about 14 minutes, between about 15 minutes and about 13 minutes, between about 1 minute and about 12 minutes, between about 1 minute and about 11 minutes, between about 1 minute and about 10 minutes, between about 1 minute and about 9 minutes, between about 1 minute and about 8 minutes, between about 1 minute and about 7 minutes, between about 1 minute and about 6 minutes, between about 1 minute and about 5 minutes, between about 1 minute and about 4 minutes, between about 1 minute and about 3 minutes, between about 1 minute and about 2 minutes, or between about 1 minute and about 90 seconds). In some examples, the first, second, and third periods of time are about the same. In other examples, the first, second, and third periods of time are not the same.

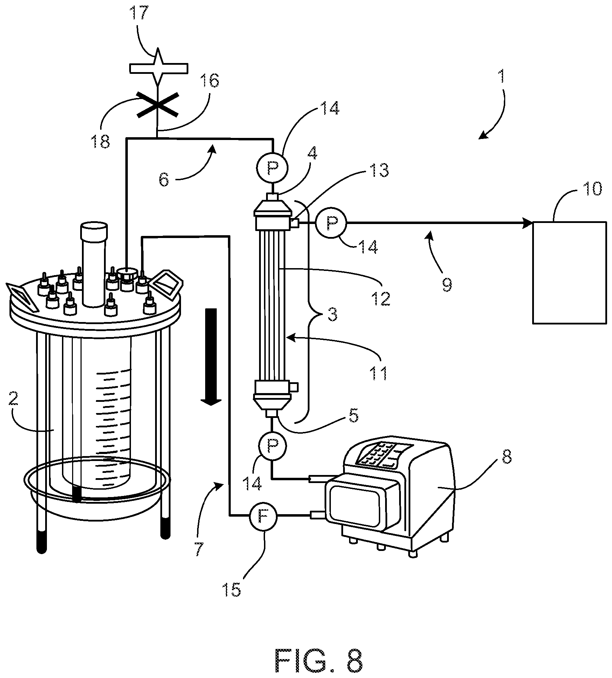

In some examples, the first flow direction in the first period of time flows the cell culture from the reservoir through the first or second conduit in which at least one pump is disposed (e.g., a single pump) is disposed, then through at least one TFF unit, then back to the reservoir through the other conduit (e.g., for a period of between about 30 seconds and about 60 minutes, between about 30 seconds and about 50 minutes, between about 30 seconds and about 40 minutes, between about 30 seconds and about 30 minutes, between about 30 seconds and about 20 minutes, between about 30 seconds and about 15 minutes, between about 30 second and about 10 minutes, or between about 30 seconds and about 5 minutes). In such examples, the flowing during the first period of time is used to equilibrate the at least one TFF unit in the system (and the at least one cross-flow filter therein). FIG. 8 is a schematic diagram showing the flowing of the cell culture in the first flow direction for the purpose of equilibrating the at least one TFF unit in the system.

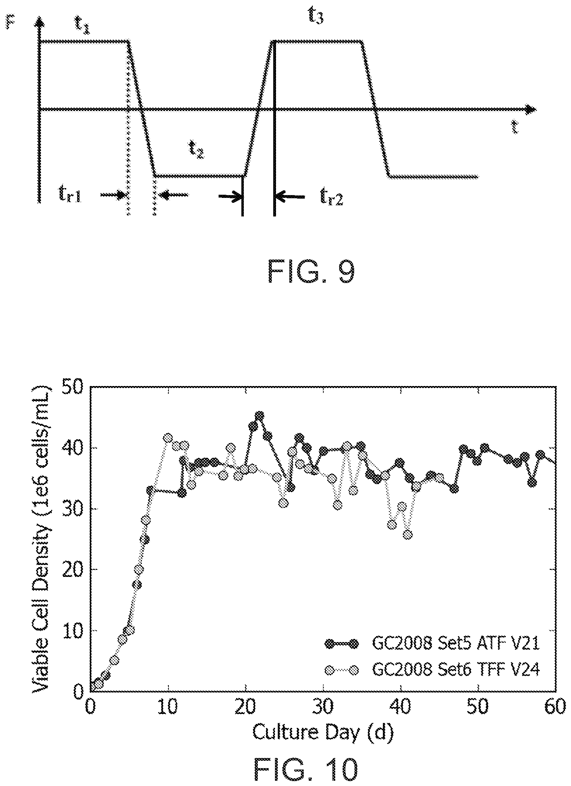

FIG. 9 shows an example of flowing of cell culture from the reservoir through the TFF unit in a first flow direction for a first period of time ($t_1$), reversing the first flow direction over a period of time ($t_{r1}$) and flowing the cell culture through the TFF unit in a second flow direction for a second period of time ($t_2$), reversing the second flow direction over a period of time ($t_{r2}$) and flowing the culture through the TFF unit in the first flow direction for a third period of time ($t_3$). For example, the $t_{r1}$ and/or the $t_{r2}$ can be between about 1 second and about 1 minute (e.g., between about 1 second and about 55 seconds, between about 1 second and about 50 seconds, between about 1 second and about 45 seconds, between about 1 second and about 40 seconds, between about 1 second and about 35 seconds, between about 1 second and about 30 seconds, between about 1 second and about 25 seconds, between about 1 second and about 20 seconds, between about 1 second and about 15 seconds, between about 1 second and about 10 seconds, between about 1 second and about 5 seconds, between about 5 seconds and about 60 seconds, between about 5 seconds and about 55 seconds, between about 5 seconds and about 50 seconds, between about 5 seconds and about 45 seconds, between about 5 seconds and about 40 seconds, between about 5 seconds and about 35 seconds, between about 5 seconds and about 30 seconds, between about 5 seconds and about 25 seconds, between about 5 seconds and about 20 seconds, between about 5 seconds and about 15 seconds, between about 5 seconds and about 10 seconds, or between about 2 second and about 10 seconds, between about 2 seconds and about 8 seconds, between about 2 seconds and about 6 seconds, or between about 2 seconds and about 4 seconds).

The flowing in the first and/or second directions (e.g., any of the first, second, and/or third time periods) can result in a flow rate of between about 0.5 L/minute to about 120 L/minute (e.g., between about 0.5 L/minute to about 115 L/minute, between about 0.5 L/minute to about 110 L/minute, between about 0.5 L/minute to about 105 L/minute, between about 0.5 L/minute to about 100 L/minute, between about 0.5 L/minute to about 95 L/minute, between about 0.5 L/minute to about 90 L/minute, between about 0.5 L/minute to about 85 L/minute, between about 0.5 L/minute to about 80 L/minute, between about 0.5 L/minute to about 75 L/minute, between about 0.5 L/minute to about 70 L/minute, between about 0.1 L/minute to about 65 L/minute, between about 0.1 L/minute to about 60 L/minute, between about 0.1 L/minute to about 55 L/minute, between about 0.1 L/minute to about 50 L/minute, between about 0.1 L/minute to about 45 L/minute, between about 0.1 L/minute to about 40 L/minute, between about 0.1 L/minute to about 35 L/minute, between about 0.1 L/minute to about 30 L/minute, between about 0.1 L/minute to about 25 L/minute, between about 0.1 L/minute to about 20 L/minute, between about 0.1 L/minute to about 15 L/minute, between about 0.1 L/minute to about 10 L/minute, or between about 0.1 L/minute to about 5 L/minute).

The single iteration of (i) flowing the cell culture in the first flow direction over the first time period and (ii) flowing the cell culture in the second flow direction over the second time period can result in an exchange fraction of between about 40% to about 95% (e.g., between about 40% to about 90%, between about 40% to about 85%, between about 40% to about 80%, between about 40% to about 75%, between about 45% to about 80%, between about 50% to about 80%, between about 55% to about 75%, between about 60% and about 85%, between about 70% and about 95%, or between about 70% and about 85%).

In the methods provided herein, the volume of cell culture in the system (with the exception of the filtrate conduit, the filtrate holding tank, and/or the biological manufacturing system) can be between about 0.1 L and about 50 L (e.g., between about 0.1 L and about 45 L, between about 0.1 L and about 40 L, between about 0.1L and about 35 L, between about 0.1 L and about 30 L, between about 0.1 L and about 25 L, between about 0.1 L and about 20 L, between about 0.1 L and about 18 L, between about 0.1 L and about 16 L, between about 0.1 L and about 14 L, between about 0.1 L and about 12 L, between about 0.1 L and about 10 L, between about 0.1 L and about 8 L, between about 0.1 L and about 6 L, between about 0.1 L and about 4 L, between about 0.1 L and about 3 L, between about 0.1 L and about 2 L, or between about 0.1 L and about 1 L). The amount of time the cell culture spends outside of the reservoir (e.g., the perfusion bioreactor) in the methods described herein can be between 5 seconds to 45 seconds (e.g., between about 5 seconds and about 40 seconds, between about 5 seconds and about 35 seconds, between about 5 seconds and about 30 seconds, between about 5 seconds and about 25 seconds, between about 5 seconds and about 20 seconds, between about 5 seconds and about 15 seconds, between about 5 seconds and about 10 seconds).

The methods described herein may produce a filtrate that does not contain a mammalian cell. The methods provided herein can also produce a filtrate that contains a secreted recombinant protein (e.g., an antibody or an antigen-binding fragment thereof, a growth factor, a cytokine, or an enzyme) from a cell culture that contains the secreted recombinant protein. In some cases, the cell culture and/or the filtrate are sterile.

The present methods can be scaled up or scaled-down to filter a larger volume of cell culture per unit of time. As can be appreciated by those skilled in the art, a larger volume of cell culture can be processed per unit of time by incorporating at least one pump with a larger pump head volume and larger tubing and/or a larger number of cross-flow filters in TFF unit(s) or a larger number of TFF units (e.g., a larger total filtration area). These changes can be implemented in the open circuit filtration system used to perform the methods described herein and can be tested to ensure that the larger scale system has one or more (e.g., two, three, four, five, six, or seven) of the following benefits: decreased external volume of cell culture (outside of the reservoir), increased exchange fraction (e.g., within the first conduit, the TFF unit, and the second conduit), decreased external residence time of cell culture (outside the reservoir), decreased sheer stress during cell culture filtration, improved cell viability in cell culture, elevated viable cell density in cell culture, and decreased filter fouling as compared to other unidirectional open circuit filtration systems (e.g., unidirectional TFF systems) or bidirectional closed circuit filtration systems (closed circuit ATFTM systems). Examples of the physical and functional parameters of three different exemplary methods and the open circuit filtration systems used to perform each method are shown in Table 2 (below).

Any of the methods described herein can be performed continuously for a period of between about 14 days and about 100 days (e.g., between about 14 days and about 90 days, between about 14 days and about 80 days, between about 14 days and about 70 days, between about 14 days and about 60 days, between about 14 days and about 50 days, between about 14 days and about 40 days, between about 14 days and about 30 days, between about 14 days and about 20 days, between about 20 days and about 100 days, between about 20 days and about 90 days, between about 20 days and about 80 days, between about 20 days and about 70 days, between about 20 days and about 60 days, between about 20 days and about 50 days, between about 20 days and about 40 days, between about 20 days and about 30 days, between about 30 days and about 100 days, between about 30 days and about 90 days, between about 30 days and about 80 days, between about 30 days and about 70 days, between about 30 days and about 60 days, between about 30 days and about 50 days, between about 30 days and about 40 days, between about 40 days and about 100 days, between about 50 days and about 90 days, between about 50 days and about 80 days, between about 50 days and about 70 days, between about 50 days and about 60 days, between about 60 days and about 100 days, between about 60 days and about 90 days, between about 60 days and about 80 days, or between about 60 days and about 70 days).

Table 2.  Parameters of Three Different Exemplary Methods and the System Used to Perform Each Method

| Filter | Working Volume (L) | Fiber Length (cm) | Filtration Area (m2) | Xv target (E6 cells/mL) | Perfusion Rate (L/d) | Exchange Rate (L/min) | External Volume (L) | Exchange Fraction (%) | External residence time (s) | Shear Rate (1/s) | XR:PR | Perfusion Flux (L/m2/hr) | Pump | Pump speed (RPM) |
|---|---|---|---|---|---|---|---|---|---|---|---|---|---|---|
| ATF4 | 10 | 50 | 0.77 | 40-80 | 20 | 3.5 | 0.55 | 78 | 12 | 716 | 252 | 1.08 | Watson-Marlow 620 w/ 16mm tubing | 68 |
| 2x ATF6 or ATF8 | 50 | 60 | 5 | 40-80 | 100 | 8 | 3.5 | 50 | 35 | 543 | 98 | 1.67 | Watson-Marlow 800 w/ 25mm tubing | 25 |
| 4x ATF10 | 500 | 60 | 40 | 40-80 | 2000 | 60 | 15 | 70 | 15 | 509 | 48 | 2.08 | Watson-Marlow 800 w/ 40mm tubing | 45 |

In some cases, the change in pressure along the filter fibers in one or more cross-flow filter(s) in the at least one TFF unit and/or the change in pressure across the filter membrane in one or more cross-flow filter(s) in the at least one TFF unit stays substantially the same (e.g., within about ± 20%, within about ±19%, within about ± 18%, within about ± 17%, within about ± 16%, within about ± 15%, within about ± 14%, within about ± 13%, within about ± 12%, within about ± 11%, within about ± 10%, within about ± 9%, within about ± 8%, within about ± 7%, within about ± 6%, within about ± 5%, within about ± 4%, within about ± 3%, within about ± 2.5%, within about ± 2.0%, within about ± 1.5%, within about ± 1.0%, or within about ± 0.5% of the initial change in pressure across the filter fibers or across the filter membrane at the beginning of the method) during the performance of the method for a period of about, e.g., between about 1 hour and about 100 days (e.g., between about 1 hour and about 95 days, between about 1 hour and about 90 days, between about 1 hour and about 90 days, between about 1 hour and about 85 days, between about 1 hour and about 80 days, between about 1 hour and about 75 days, between about 1 hour and about 70 days, between about 1 hour and about 65 days, between about 1 hour and about 60 days, between about 1 hour and about 55 days, between about 1 hour and about 50 days, between about 1 hour and about 45 days, between about 1 hour and about 40 days, between about 1 hour and about 35 days, between about 1 hour and about 30 days, between about 1 hour and about 25 days, between about 1 hour and about 20 days, between about 1 hour and about 15 days, between about 1 hour and about 10 days, between about 1 hour and about 5 days, between about 1 day and about 100 days, between about 1 day and about 90 days, between about 1 day and about 85 days, between about 1 day and about 80 days, between about 1 day and about 75 days, between about 1 day and about 70 days, between about 1 day and about 65 days, between about 1 day and about 60 days, between about 1 day and about 55 days, between about 1 day and about 50 days, between about 1 day and about 45 days, between about 1 day and about 40 days, between about 1 day and about 35 days, between about 1 day and about 30 days, between about 1 day and about 25 days, between about 1 day and about 20 days, between about 1 day and about 15 days, between about 1 day and about 10 days, between about 5 days and about 100 days, between about 5 days and about 95 days, between about 5 days and about 90 days, between about 5 days and about 85 days, between about 5 days and about 80 days, between about 5 days and about 75 days, between about 5 days and about 70 days, between about 5 days and about 65 days, between about 5 days and about 60 days, between about 5 days and about 55 days, between about 5 days and about 50 days, between about 5 days and about 45 days, between about 5 days and about 40 days, between about 5 days and about 35 days, between about 5 days and about 30 days, between about 5 days and about 25 days, between about 5 days and about 20 days, between about 5 days and about 15 days, between about 5 days and about 10 days, between about 10 days and about 100 days, between about 10 days and about 95 days, between about 10 days and about 90 days, between about 10 days and about 85 days, between about 10 days and about 80 days, between about 10 days and about 75 days, between about 10 days and about 70 days, between about 10 days and about 65 days, between about 10 days and about 60 days, between about 10 days and about 55 days, between about 10 days and about 50 days, between about 10 days and about 45 days, between about 10 days and about 40 days, between about 10 days and about 35 days, between about 10 days and about 30 days, between about 10 days and about 25 days, between about 10 days and about 20 days, between about 15 days and about 100 days, between about 15 days and about 95 days, between about 15 days and about 90 days, between about 15 days and about 85 days, between about 15 days and about 80 days, between about 15 days and about 75 days, between about 15 days and about 70 days, between about 15 days and about 65 days, between about 15 days and about 60 days, between about 15 days and about 55 days, between about 15 days and about 50 days, between about 15 days and about 45 days, between about 15 days and about 40 days, between about 15 days and about 35 days, between about 15 days and about 30 days, between about 15 days and about 25 days, or between about 15 days and about 20 days). A significant increase in the change in pressure across the filter fiber or the filter membrane indicates fouling of the at least one cross-flow filter in at least one TFF unit in the system.

***Incubating** the **Cell Culture in** the Reservoir*

Some cases further include incubating the cell culture in the reservoir (e.g., perfusion bioreactor) under conditions that allow for the mammalian cell to secrete a recombinant protein into the tissue culture medium. For example, the cell culture in the reservoir can be incubated at a temperature of about 32 °C to about 39 °C. Skilled practitioners will appreciate that the temperature can be changed at specific time point(s) during the incubation (e.g., on an hourly or daily basis). For example, the temperature can be changed or shifted (e.g., increased or decreased) at about one day, two days, three days, four days, five days, six days, seven days, eight days, nine days, ten days, eleven days, twelve days, fourteen days, fifteen days, sixteen days, seventeen days, eighteen days, nineteen days, or about twenty days or more after placement of the cell culture into the reservoir). For example, the temperature can be shifted upwards (e.g., a change of up to or about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10.0 °C). For example, the temperature can be shifted downwards (e.g., a change of up to or about 0.1, 0.2, 0.3, 0.4, 0.5, 0.6, 0.7, 0.8, 0.9, 1.0, 1.5, 2.0, 2.5, 3.0, 3.5, 4.0, 4.5, 5.0, 5.5, 6.0, 6.5, 7.0, 7.5, 8.0, 8.5, 9.0, 9.5, or 10 °C). The incubating of the cell culture in a reservoir can also be performed in an atmosphere containing at most or about 1% to 15% $CO_2$ (e.g., at most or about 14% $CO_2$, 12% $CO_2$, 10% $CO_2$, 8% $CO_2$, 6% $CO_2$, 5% $CO_2$, 4% $CO_2$, 3% $CO_2$, 2% $CO_2$, or at most or about 1% $CO_2$). Moreover, any of the methods described herein can include incubating the cell culture in a humidified atmosphere (e.g., at least or about 20%, 30%, 40%, 50%, 60%, 70%, 85%, 80%, 85%, 90%, or at least or about 95% humidity, or about 100% humidity).

The incubating of the cell culture in a reservoir (e.g., a perfusion bioreactor) during the reiteration of the first, second, and third time periods, can include a step of adding a volume of liquid culture medium to the bioreactor. For example, the addition of the volume of liquid culture medium to the bioreactor can counterbalance the loss of liquid culture medium that leaves the system as filtrate. The adding of liquid culture medium to the reservoir can be performed continuously or periodically (e.g., once every third day, once every other day, once a day, twice a day, three times a day, four times a day, five times a day, or more than five times a day), or any combination thereof. The volume of liquid culture medium added to the reservoir can in some instances be performed such that the starting volume of cell culture in the system (excluding the volume of the filtrate present in the filtrate conduit and the filtrate holding tank) is approximately the same over each 24-hour period or over the entire period that the method is performed. As is known in the art, the rate at which the liquid culture medium is removed from the system as filtrate (volume/unit of time) and the rate at which the volume of the liquid culture medium is added to the reservoir (volume/unit of time) can be varied. The rate at which the liquid culture medium is removed from the system as filtrate (volume/unit of time) and the rate at which the volume of the liquid culture medium is added (volume/unit of time) can be about the same or can be different.

Alternatively, the volume removed from the system as filtrate and the volume added to the reservoir can change (e.g., gradually increase) over each 24-hour period (or alternatively, an incremental time period of between 0.1 hour and about 24 hours or an incremental time period of greater than 24 hours) during the performance of the method. For example the volume of liquid culture medium removed from the system as filtrate and the volume of the liquid culture medium added within each 24-hour period (or alternatively, an incremental time period of between about 1 hour and above 24 hours or an incremental time period of greater than 24 hours) over the performance of the method can be increased (e.g., gradually or through staggered increments), e.g., from a volume that is between 0.5% to about 20% of the reservoir volume or the total volume of the cell culture at the beginning of the performance of the method to about 25% to about 150% of the volume of the reservoir or the total volume of the cell culture at the beginning of the performance of the method. As can be appreciated by one skilled in the art, within each 24-hour period, the volume removed from the system as filtrate and the volume added to the reservoir is preferably about 100% to about 400% (e.g., between about 100% and about 350%, between about 100% and about 300%, between about 100% and about 250%, between about 100% and about 200%, between about 100% and about 150%, between about 150% and about 400%, between about 150% and about 350%, between about 150% and about 300%, between about 150% and about 250%, between about 150% and about 200%, between about 200% and about 400%, between about 200% and about 350%, between about 200% and about 300%, or between about 200% and about 250%) of volume of the reservoir or the total volume of the cell culture at the beginning of the performance of the method.

Skilled practitioners will appreciate that the liquid culture medium removed from the system as filtrate and the liquid culture medium added to the reservoir can be the same type of media. In other instances, the liquid culture medium removed from the system as filtrate and the liquid culture medium added to the reservoir can be substantially different. The volume of the liquid culture medium can be added to the manually or using an automated system, e.g., by perfusion pump.

## Isolating the Recombinant Protein from the Filtrate

Any of the methods described herein can further include a step of isolating the secreted recombinant protein (e.g., any of the recombinant proteins described herein) from the filtrate. Many methods for isolating a polypeptide (e.g., a secreted polypeptide) from a fluid are known in the art. For example, methods for isolating a recombinant protein can include one or more steps of: capturing, purifying, polishing, and/or filtering a fluid containing the recombinant protein. As is well-known in the art, the specific methods used to isolate a recombinant protein will depend on the biophysical properties of the recombinant protein. For example, a recombinant antibody can be purified using, in part, a step of capturing the antibody using a protein A resin.

In some examples, a recombinant protein present in the filtrate is isolated using an integrated and continuous process that includes isolating through at least one multi-column chromatography system (MCCS) (e.g., any of the one or more MCCSs described herein). The integrated and continuous process can be performed using any of the exemplary biological manufacturing systems described herein. Exemplary integrated and continuous processes for isolating a recombinant protein and biological manufacturing systems to be used in such processes are described in the art.

The resulting isolated recombinant protein can be at least or about 50% pure by weight, e.g., at least or about 55% pure by weight, at least 60% pure by weight, at least 65% pure by weight, at least 70% pure by weight, at least 75% pure by weight, at least 80% pure by weight, at least 85% pure by weight, at least 90% pure by weight, at least 95% pure by weight, at least 96% pure by weight, at least 97% pure by weight, at least 98% pure by weight, or at least or about 99% pure by weight, or greater than 99% pure by weight.

Some methods further include a step of formulating a therapeutic drug substance by mixing the isolated recombinant protein with a pharmaceutically acceptable excipient or buffer. The mixing can be performed by mixing a fluid containing the isolated recombinant protein with a buffered solution. In other examples, the mixing can be performed by adding a solid buffering agent to a fluid containing the isolated recombinant protein with a buffered solution. Another form of mixing, as encompassed herein, is dissolving a solid composition (e.g., a lyophilized powder or cake) containing the isolated recombinant protein with a buffered solution (e.g., injectable sterile saline). The therapeutic drug substance can be formulated for any route of administration known in the art (e.g., oral administration, intravenous administration, intaarterial administration, intramuscular administration, intraperitoneal administration, subcutaneous administration, intrathecal administration, or inhalation).

## EXAMPLES

The invention is further described in the following examples, which do not limit the scope of the invention described in the claims.

## Example 1. Comparison of Processing Achieved by Open Circuit Filtration Systems Provided Herein Versus Processing Achieved by ATF™ (Refine Technology)

A set of experiments was performed to compare the cell culture processing achieved by an open circuit filtration system provided herein to the cell culture processing achieved by ATF™ (Refine Technology) (a closed circuit alternating flow tangential filtering system). The device used to perform these experiments is generally depicted in FIG. 5. Specifically, the reservoir used in the open circuit filtration system is a Broadly-James 15L bioreactor, the first conduit and the second conduit are biocompatible, weldable transfer tubing with an internal diameter of 0.5 inch, the TFF unit contains a single tubular cross-flow filter (composed of polyethersulfone fibers with a length of 30 cm and an internal diameter of 1 mm, and having an average pore size of 0.2 $\mu$mm, a fiber density of 830 fibers/filter, and a filtration area of 0.77 m$^2$), at least one pump is a single Watson-Marlow peristaltic pump capable of flowing a fluid in the first and second flow directions, with a pump head volume of between 50 mL to 100 mL with twin channel GORE Sta-Pure tubing having an internal diameter of 16 mm and a wall diameter of 4 mm.

### Materials and Methods

A summary of the experimental parameters used for comparison of the processing achieved by the presently provided open circuit filtration systems and the ATF™ by Refine Technology is summarized in Tables 3 and 4 (below). A further detailed summary of the methods used to perform these experiments is provided below.

Table 3. Experimental Parameters

| Parameter | Detailed Description | |
| --- | --- | --- |
| | TFF | ATF4 |
| Cell line | GC2008 clone A61, High Density bank "GC2008 A61 HD WAVE," 45 x $10^7$ cells/vial | |
| Media | CD CHO with glutamine | |
| Bioreactors | Broadly James 15 L bioreactor | |
| Working Volume | 10 L | |
| Bioreactor inoculum | Shake flask seed train | |
| Inoculation Density | 0.5 - 1x$10^6$ cells/mL | |
| Cell density target | Allow to reach 40x $10^6$ cells/mL with 2 Reactor Volume (RV)/day, and bleed to maintain | |
| Cell specific perfusion rate | 0.05 nL/cell-d | |
| Biomass Removal (as needed) | If capacitance (Aber) vs. cell density correlation is good, use capacitance to control bleed rate. Alternatively, use $O_2$ sparge | |
| Temperature | 37 °C | |
| Agitation | 120 RPM | |
| DO | $\geq$ 40% | |
| Base | 1M $Na_2CO_3$ (sodium carbonate) | |
| Antifoam | Invitrogen Foam Away 3% Simethicone (30,000 PPM) Working stock: 3000PPM (dilute in WFI) | |
| pCO2 | <120 mmHg, sparge with $N_2$ if >120 mmHg | |
| pH | 6.95 $\pm$ 0.1 | |
| Gas addition | Sparge: Oxygen, $CO_2$ (as needed), $N_2$ (as needed) Overlay: Air at 100 ccpm | |
| O2 Sparger | 20 $\mu$m sintered | |
| N2 Sparger | 1 mm Drilled hole | |
| Cell Separation Device | TFF with Watson-Marlow 620Du peristaltic pump with 620L pumphead, 16mm ID Gore sta-pure tubing ATF4 filter (0.2 $\mu$m) | Refine ATF4 |
| ATF/TFF exchange rate | 3.5 L/min (65-70 rpm), reverse every 1 min | 3.5 L/min, reverse every 7 seconds |

Table 4. Comparison of Parameters

| | Exchange rate (L/min) | Transfer Tubing ID (in) | External volume (L) | Exchange Fraction (%) | External residence time (s) | Pump RPM | Shear rate (1/s) | Exchange rate: Perfusion Rate | Pump Inversion time (s) |
|---|---|---|---|---|---|---|---|---|---|
| ATF4 | 3.5 | 0.375 | 0.756 | 19 | 71 | N/A | 716 | 252 | 7 |
| TFF | 3.5 | 0.500 | 0.550 | 78 | 12.1 | 68.5 | 716 | 252 | 60 |

The conditions used to run the perfusion bioreactor are listed in Table 3. The bioreactors were maintained at 40 x $10^6$ cells/mL with 10 L working volume and 2 reactor volume/day replacement with CD-CHO culture medium. The tested open circuit filtration system provided herein contained the same filter and housing as ATF4, but used a Watson-Marlow peristaltic pump 620 Du with a pump head volume of between 50 mL to 100 mL as a culture recirculation pump to reversibly flow the cell culture through the system (shown in FIG. 5) and an open circuit system (rather than a closed system used in ATF4). The ATF4 bioreactor perfusion rate was changed from 2 reactor volume/day to 1 reactor volume/day on day 20 of culture, whereas the tested open circuit filtration system provided herein was changed from a perfusion rate of 2 reactor volumes/day to 1 reactor volume/day on day 32, and 10% Efficient Feed B (Gibco, Invitrogen) was also supplemented.

*Results*

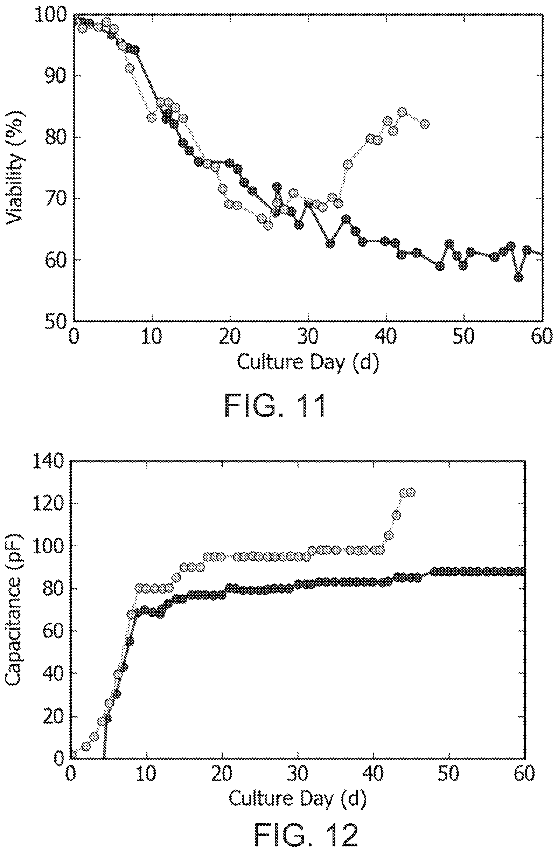

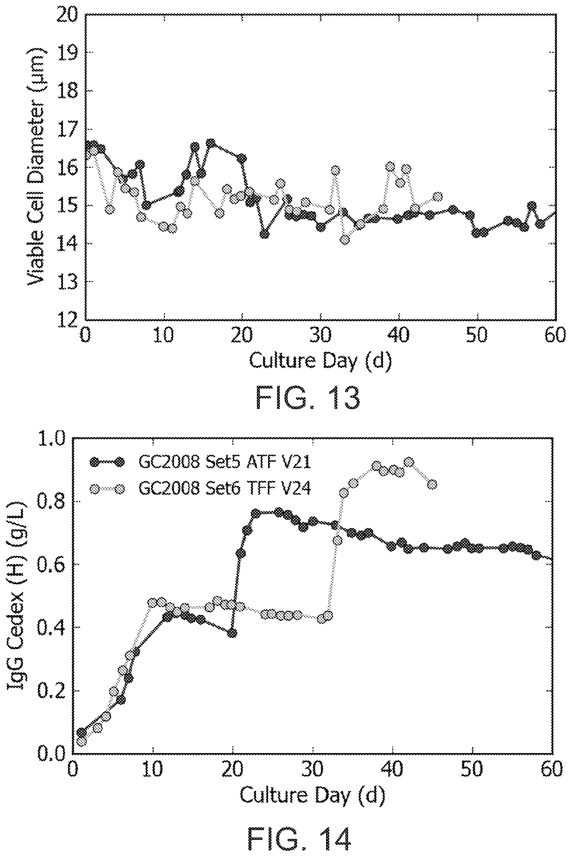

The tested open circuit filtration system provided herein reached a viable cell density of 40 x $10^6$ cells/mL at day 9 and 10, and reached a cell density of 40 x $10^6$ cells/mL earlier than the corresponding ATF system (FIG. 10). The percentage of viable cells of the tested open circuit filtration system provided herein was about 90% once the culture reached 40 x $10^6$ cells/mL, and continued to decrease until stabilized at 70% over three weeks (FIG. 11). The capacitance of the cell culture in the tested open circuit filtration system provided herein was elevated as compared to the cell culture in the ATF system (FIG. 12), and the mean viable cell diameter from the cell culture of the tested open circuit filtration system provided herein and the cell culture of the ATF system were similar (FIG. 13).

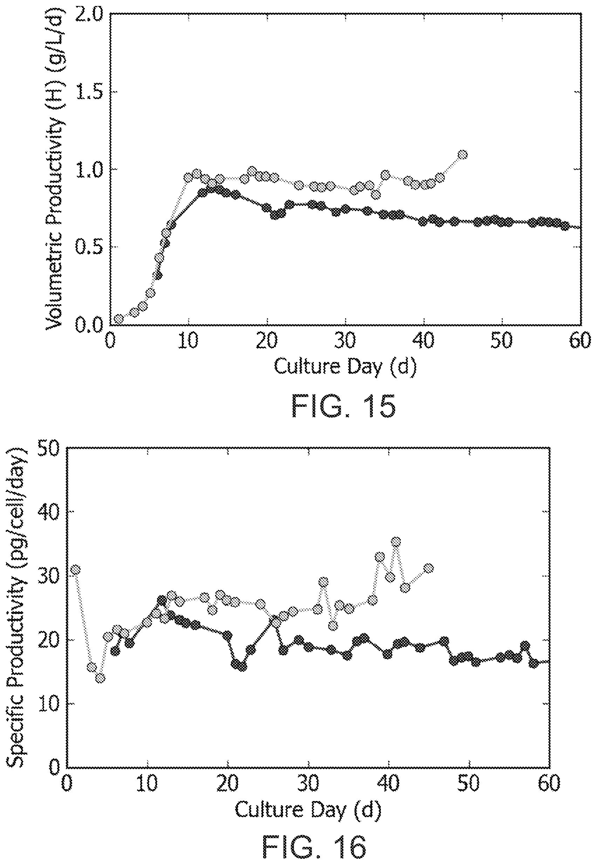

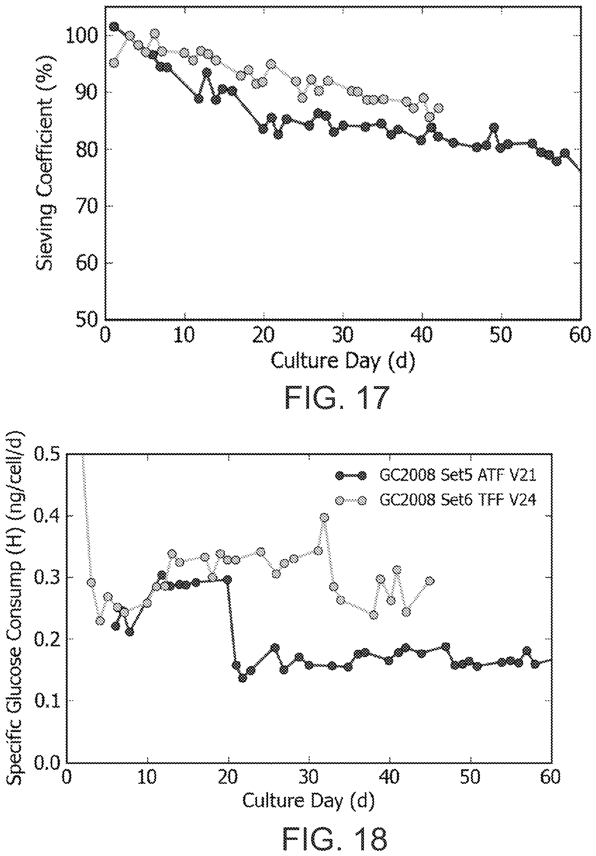

The productivity profiles of the cell cultures in the open circuit filtration system provided herein and the cell culture of the ATF system are shown in FIG. 14, FIG. 15, FIG. 16, and FIG. 17. The concentration of IgG produced by the cell culture in the open circuit filtration system provided herein was increased at later time points as compared to the ATF system (FIG. 14). The volumetric productivity and specific productivity of the cell culture in the open circuit filtration system provided herein was increased as compared to the cell culture in the ATF system (FIG. 15 and FIG. 16, respectively). The sieving coefficient of the cell culture in the tested open circuit filtration system provided herein remained at about 90% after three weeks of culture, and was greater than the sieving coefficient of the cell culture in the ATF system (FIG. 17).

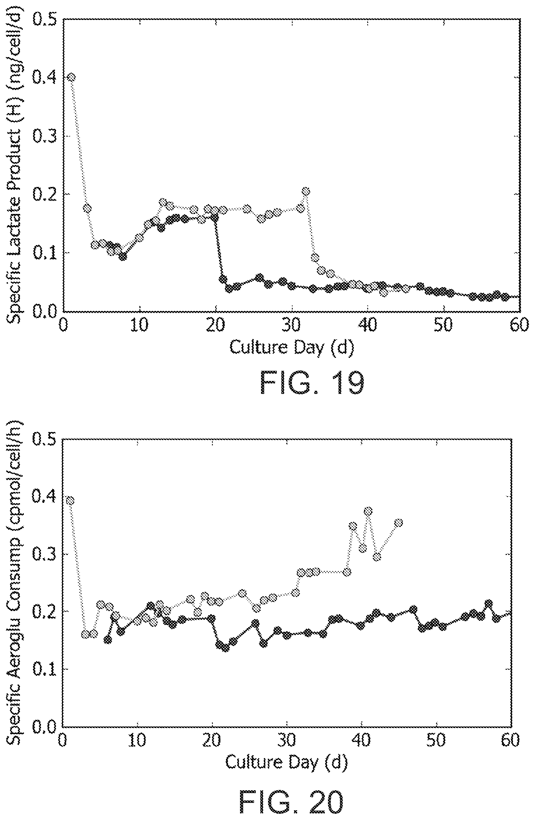

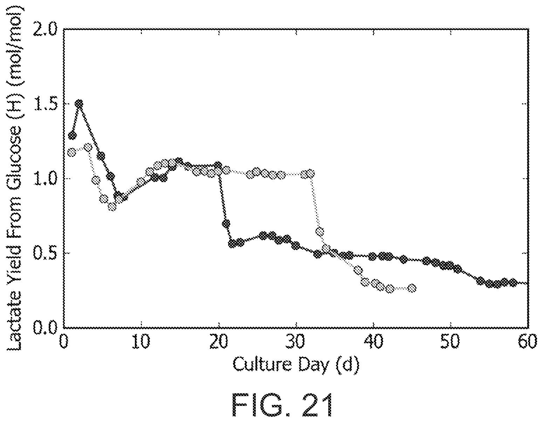

The glucose and lactate production profiles of each tested system are shown in FIG. 18, FIG. 19, FIG. 20, and FIG. 21. The specific glucose consumption rate and the specific lactate production rate of the cell culture in the tested open circuit filtration system provided herein was greater than the specific glucose consumption rate and the specific lactate production rate of the cell culture in the ATF system (FIG. 18 and FIG. 19, respectively). In addition, the specific aerobic glucose consumption rate and the lactate yield from glucose was higher in the cell culture in the tested open circuit filtration system provided herein than the specific aerobic glucose consumption rate and the lactate yield from glucose in the cell culture in the ATF system (FIG. 20 and FIG. 21, respectively).

These data indicated that the presently provided open circuit filtration systems provide for a cell culture with improved or comparable cell culture properties such as increased or comparable capacitance, increased or comparable volumetric and specific productivity, increased or comparable sieving coefficient, and increased or comparable specific glucose consumption as compared to another closed circuit tangential filtration system (ATF™ system by Refine Technology).

**Example 2. Viable Cell Density Observed in Open Circuit Filtration Systems**

An experiment is performed to determine the highest viable cell densities achieved using an open circuit filtration system provided herein, and optionally, comparing the determined viable cell densities to the viable cell densities achieved using ATF™ (Refine Technology) (a closed circuit alternating flow tangential filtering system), under similar conditions. The device to be used in these experiments is generally depicted in FIG. 5. Specifically, the reservoir to be used in the open circuit filtration system is a Broadly-James 15L bioreactor, the first conduit and the second conduit are biocompatible, weldable transfer tubing with an internal diameter of 0.5 inch, the TFF unit contains a single tubular cross-flow filter (composed of polyethersulfone fibers with a length of 30 cm and an internal diameter of 1 mm, and having an average pore size of 0.2 μmm, a fiber density of 830 fibers/filter, and a filtration area of 0.77 $m^2$), the at least one pump is a single Watson-Marlow peristaltic pump capable of flowing a fluid in the first and second flow directions, with a pump head volume of between 50 mL to 100 mL with twin channel GORE Sta-Pure tubing having an internal diameter of 16 mm and a wall diameter of 4 mm.

*Materials and Methods*

A summary of the experimental parameters for determining the highest cell densities that can be achieved using the presently provided open circuit filtration systems (and optionally the ATF™ by Refine Technology) is shown in Table 5. A further detailed summary of the methods to be used in these experiments is provided below.

Table 5. Experimental Parameters

| Parameter | Detailed Description | |
|---|---|---|
| | TFF | ATF4 |
| Cell line | GC2008 clone A61, HD bank "GC2008 A61 HD WAVE," 45 x 10$^7$ cells/vial | |
| Media | CD CHO with glutamine | |
| Bioreactors | Broadly James 15 L bioreactor | |
| Working Volume | 10 L | |
| Bioreactor inoculum | Shake flask seed train | |
| Inoculation Density | 0.5 - 1x10$^6$ cells/mL | |
| Cell density | Allow cells to continue to grow with increasing perfusion rate in order to match CSPR of 0.05 nL/cell-d, with no or low constant bleed to maintain cell density | |
| Cell specific perfusion rate (CSPR) | 0.05 nL/cell-d | |
| Temperature | 37 °C | |
| Agitation | 120 RPM | |
| DO | $\geq$ 40% | |
| Base | 1M $Na_2CO_3$ (sodium carbonate) | |
| Antifoam | Invitrogen Foam Away 3% Simethicone (30,000 PPM) | |
| | Working stock: 3000PPM (dilute in WFI) | |
| pCO2 | <120 mmHg, sparge with $N_2$ if >120 mmHg | |
| pH | 6.95 $\pm$ 0.1 | |
| Gas addition | Sparge: Oxygen, $CO_2$ (as needed), $N_2$ (as needed) Overlay: Air at 100 ccpm | |
| O2 Sparger | 20 $\mu$m sintered | |
| N2 Sparger | 1 mm Drilled hole | |
| Cell Separation Device | TFF with Watson-Marlow 620Du peristaltic pump with 620L pumphead, 16mm ID Gore sta-pure tubing ATF4 filter (0.2 $\mu$m) | Refine ATF4 |
| ATF/TFF exchange rate | 3.5 L/min (65-70 rpm), reverse every 1 min | 3.5 L/min, reverse every 7 seconds |

The conditions to be used to run the perfusion bioreactor are listed in Table 5. The cells are allowed to growth in the bioreactors, with a 10-L working volume, and a sufficient replacement with CD-CHO culture medium to maintain cell specific perfusion rate of 0.05 nL/cell-d. The open circuit filtration system contains the same filter and housing as ATF4, but uses a Watson-Marlow peristaltic pump 620 Du with a pump head volume of between 50 mL to 100 mLas a culture recirculation pump to reversibly flow the cell culture through the system (shown in FIG. 5) and an open circuit system (rather than a closed system used in ATF4). The viable cell density of the cell culture is determined once a day for the duration of the cell culture process run.

**Claims**

1. A method of processing a cell culture, the method comprising:

(a) providing an open circuit filtration system comprising a bioreactor comprising a cell culture, a tangential flow filtration (TFF) unit having first and second inlets, a first conduit in fluid communication between the bioreactor and the TFF unit first inlet, and a second conduit in fluid communication between the bioreactor and the TFF unit second inlet, and at least one pump disposed within the system for flowing fluid through the system, wherein the system is configured such that fluid can be flowed reversibly through the system from or to the bioreactor and through the first and second conduits and the TFF unit via the at least one pump, and filtrate can be collected from the TFF unit;

(b) flowing cell culture from the bioreactor through the first and second conduits and the TFF unit in a first flow direction for a first period of time,

(c) reversing the first flow direction and flowing the cell culture through the first and second conduits and the TFF unit in a second flow direction for a second period of time;

(d) reversing the second flow direction and flowing the culture through the first and second conduits and the TFF unit in the first flow direction for a third period of time;

(e) repeating steps (c) - (d) at least two times; and

(f) collecting the filtrate.

2. The method of claim 1, wherein the TFF unit comprises a single cross-flow filter, e.g., a tubular cross-flow filter.

3. The method of claim 1, wherein the TFF unit comprises two or more cross-flow filters.

4. The method of claim 1, wherein the system comprises one or more additional TFF units disposed in the first conduit, the second conduit, or both, and optionally, the at least one pump is disposed in the system between any two TFF units.

5. The method of claim 1, wherein the at least one pump is disposed in:

the first conduit or the second conduit, or both; or
the bioreactor and proximal to the first or second fluid conduit.

6. The method of any one of claims 1 to 5, wherein the at least one pump is a low turbulence pump (LTP), e.g., a peristaltic pump.

7. The method of claim 6, wherein the system comprises:

a first and a second LTP, wherein the first LTP flows the cell culture in the first direction and the second LTP flows the cell culture in the second direction; or
a single LTP, where the single LTP flows the cell culture in the first direction during the first and third time periods and flows the cell culture in the second direction during the second time period.

8. The method of claim 1, wherein the filtrate does not contain a mammalian cell.

9. The method of claim 1, wherein the cell culture contains a secreted recombinant protein, e.g., an antibody or antigen-binding fragment thereof, a growth factor, a cytokine, or an enzyme, and the filtrate contains the secreted recombinant protein.

10. An open circuit filtration system comprising (1) a bioreactor, (2) a tangential flow filtration (TFF) unit having first and second inlets, (3) a first conduit in fluid communication between the bioreactor and the TFF unit first inlet, (4) a second conduit in fluid communication between the bioreactor and the TFF unit second inlet, and (5) at least one pump disposed within the system, wherein actuating the at least one pump flows fluid reversibly through the system from the bioreactor, through the first conduit, the TFF unit, the second conduit, and back to the reservoir, and optionally, further comprising (6) a filtrate holding tank and a filtrate conduit in fluid communication between the TFF unit and the filtrate holding tank.

11. The open circuit filtration system of claim 10, wherein the system comprises one or more additional TFF units disposed in the first conduit, the second conduit, or both.

12. The open circuit filtration system of claim 10, wherein the at least one pump is:

disposed in the first conduit or the second conduit, or both; or disposed in the bioreactor and proximal to the first or second fluid conduit.

**13.** The open circuit filtration system of any one of claims 10 to 12, wherein the at least one pump is a low turbulence pump (LTP), e.g., a peristaltic pump.

**14.** The open circuit filtration system of claim 13, wherein the system comprises:

a first and a second LTP, wherein the first LTP is adapted to flow the cell culture in a first flow direction and the second LTP is adapted to reverse the first flow direction and flow the cell culture in a second flow direction; or a single LTP adapted to reversibly flow the cell culture in a first and second flow directions.

**15.** The method according to any one of claims 1 to 9, wherein the external residence time of the cell culture is between 1 second and 20 seconds.

**Patentansprüche**

**1.** Verfahren zum Prozessieren einer Zellkultur, das Verfahren umfassend:

(a) Bereitstellen eines Filtrationssystems mit offenem Kreislauf, umfassend einen Bioreaktor, der eine Zellkultur umfasst, eine Tangentialstromfiltrationseinheit (TFF-Einheit), die einen ersten und einen zweiten Einlass aufweist, eine erste Leitung in Fluidverbindung zwischen dem Bioreaktor und dem ersten Einlass der TFF-Einheit, und eine zweite Leitung in Fluidverbindung zwischen dem Bioreaktor und dem zweiten Einlass der TFF-Einheit, und mindestens eine Pumpe, die in dem System angeordnet ist, damit Fluid durch das System strömt, wobei das System konfiguriert ist, sodass Fluid umkehrbar von oder zu dem Bioreaktor und durch die erste und die zweite Leitung und die TFF-Einheit über die mindestens eine Pumpe durch das System strömen kann und Filtrat von der TFF-Einheit gesammelt werden kann;
(b) Strömen von Zellkultur aus dem Bioreaktor durch die erste und die zweite Leitung und die TFF-Einheit in einer ersten Strömungsrichtung über eine erste Zeitperiode,
(c) Umkehren der ersten Strömungsrichtung und Strömen der Zellkultur durch die erste und die zweite Leitung und die TFF-Einheit in einer zweiten Strömungsrichtung über eine zweite Zeitperiode;
(d) Umkehren der zweiten Strömungsrichtung und Strömen der Zellkultur durch die erste und die zweite Leitung und die TFF-Einheit in der zweiten Strömungsrichtung über eine dritte Zeitperiode;
(e) Wiederholen von Schritten (c) - (d) mindestens zwei Mal; und
(f) Sammeln des Filtrats.

**2.** Verfahren nach Anspruch 1, wobei die TFF-Einheit einen einzelnen Querstromfilter, z. B. einen rohrförmigen Querstromfilter, umfasst.

**3.** Verfahren nach Anspruch 1, wobei die TFF-Einheit zwei oder mehrere Querstromfilter umfasst.

**4.** Verfahren nach Anspruch 1, wobei das System eine oder mehrere zusätzliche TFF-Einheiten umfasst, die in der ersten Leitung, der zweiten Leitung oder in beiden angeordnet sind, und optional die mindestens eine Pumpe in dem System zwischen zwei beliebigen TFF-Einheiten angeordnet ist.

**5.** Verfahren nach Anspruch 1, wobei die mindestens eine Pumpe in Folgendem angeordnet ist:

in der ersten Leitung oder der zweiten Leitung, oder beiden; oder
in dem Bioreaktor und proximal der ersten oder zweiten Fluidleitung.

**6.** Verfahren nach einem der Ansprüche 1 bis 5, wobei die mindestens eine Pumpe eine Pumpe niedriger Turbulenz (LTP) ist, z. B. eine Peristaltikpumpe.

**7.** Verfahren nach Anspruch 6, wobei das System Folgendes umfasst:

eine erste und eine zweite LTP, wobei die erste LTP die Zellkultur in die erste Richtung strömen lässt und die zweite LTP die Zellkultur in die zweite Richtung strömen lässt; oder
eine einzelne LTP, wobei die einzelne LTP die Zellkultur während der ersten und der dritten Zeitperiode in die erste Richtung strömen lässt und die Zellkultur während der zweiten Zeitperiode in die zweite Richtung strömen lässt.

8. Verfahren nach Anspruch 1, wobei das Filtrat keine Säugetierzelle enthält.

9. Verfahren nach Anspruch 1, wobei die Zellkultur ein sekretiertes rekombinantes Protein enthält, z. B. einen Antikörper oder ein antigenbindendes Fragment davon, einen Wachstumsfaktor, ein Zytokin oder ein Enzym, und das Filtrat das sezernierte rekombinante Protein enthält.

10. Filtrationssystem mit offenem Kreislauf, umfassend (1) einen Bioreaktor, (2) eine Tangentialstromfiltrationseinheit (TFF-Einheit), die einen ersten und einen zweiten Einlass aufweist, (3) eine erste Leitung in Fluidverbindung zwischen dem Bioreaktor und dem ersten Einlass der TFF-Einheit, (4) eine zweite Leitung in Fluidverbindung zwischen dem Bioreaktor und dem zweiten Einlass der TFF-Einheit, und (5) mindestens eine Pumpe, die in dem System angeordnet ist, wobei ein Betätigen der mindestens einen Pumpe Fluid umkehrbar durch das System von dem Bioreaktor durch die erste Leitung, die TFF-Einheit, die zweite Leitung und zurück zu dem Behälter strömen lässt, und optional ferner umfassend (6) einen Filtratauffangtank und eine Filtratleitung in Fluidverbindung zwischen der TFF-Einheit und dem Filtratauffangtank.

11. Filtrationssystem mit offenem Kreislauf nach Anspruch 10, wobei das System eine oder mehrere zusätzliche TFF-Einheiten umfasst, die in der ersten Leitung, in der zweiten Leitung oder in beiden angeordnet sind.

12. Filtrationssystem mit offenem Kreislauf nach Anspruch 10, wobei die mindestens eine Pumpe wie folgt ist: in der ersten Leitung oder der zweiten Leitung oder in beiden angeordnet; oder in dem Bioreaktor und proximal zu der ersten oder zweiten Fluidleitung angeordnet ist.

13. Filtrationssystem mit offenem Kreislauf nach einem der Ansprüche 10 bis 12, wobei die mindestens eine Pumpe eine Pumpe niedriger Turbulenz (LTP) ist, z. B. eine Peristaltikpumpe.

14. Filtersystem mit offenem Kreislauf nach Anspruch 13, wobei das System Folgendes umfasst:

eine erste und eine zweite LTP, wobei die erste LTP angepasst ist, um die Zellkultur in eine erste Strömungsrichtung strömen zu lassen und die zweite LTP angepasst ist, um die erste Strömungsrichtung umzukehren und die Zellkultur in eine zweite Strömungsrichtung strömen zu lassen; oder eine einzelne LTP, die angepasst ist, um die Zellkultur umkehrbar in eine erste und eine zweite Strömungsrichtung strömen zu lassen.

15. Verfahren nach einem der Ansprüche 1 bis 9, wobei die externe Verweilzeit der Zellkultur zwischen 1 Sekunde und 20 Sekunden ist.

**Revendications**

1. Procédé de traitement d'une culture cellulaire, le procédé comprenant :

(a) la fourniture d'un système de filtration à circuit ouvert comprenant un bioréacteur comprenant une culture cellulaire, une unité de filtration à flux tangentiel (TFF) présentant une première et une deuxième entrées, un premier conduit en communication fluidique entre le bioréacteur et la première entrée de l'unité TFF, et un deuxième conduit en communication fluidique entre le bioréacteur et la deuxième entrée de l'unité TFF, et au moins une pompe disposée dans le système pour permettre l'écoulement de fluide à travers le système, dans lequel le système est configuré de sorte que le fluide puisse s'écouler de manière réversible à travers le système depuis ou vers le bioréacteur et à travers le premier et le deuxième conduits et l'unité TFF par le biais de l'au moins une pompe, et le filtrat peut être collecté à partir de l'unité TFF ;
(b) l'écoulement d'une culture cellulaire du bioréacteur à travers le premier et le deuxième conduits et l'unité TFF dans une première direction d'écoulement pendant un premier laps de temps,
(c) l'inversion du premier sens de direction et l'écoulement de la culture cellulaire à travers le premier et le deuxième conduits et l'unité TFF dans une deuxième direction d'écoulement pendant un deuxième laps de temps ;
(d) l'inversion de la deuxième direction d'écoulement et l'écoulement de la culture à travers le premier et le deuxième conduits et l'unité TFF dans la première direction d'écoulement pendant un troisième laps de temps ;
(e) la répétition des étapes (c) - (d) au moins deux fois ; et
(f) la collecte du filtrat.

**2.** Procédé selon la revendication 1, dans lequel l'unité TFF comprend un filtre à écoulement croisé unique, par exemple un filtre à écoulement croisé tubulaire.

**3.** Procédé selon la revendication 1, dans lequel l'unité TFF comprend deux filtres à écoulement croisé ou plus.

**4.** Procédé selon la revendication 1, dans lequel le système comprend une ou plusieurs unités TFF supplémentaires disposées dans le premier conduit, le deuxième conduit ou les deux, et éventuellement l'au moins une pompe est disposée dans le système entre n'importe laquelle des deux unités TFF.

**5.** Procédé selon la revendication 1, dans lequel l'au moins une pompe est disposée dans :

le premier conduit ou le deuxième conduit, ou les deux ; ou
le bioréacteur et près du premier ou du deuxième conduit de fluide.

**6.** Procédé selon l'une quelconque des revendications 1 à 5, dans lequel l'au moins une pompe est une pompe à faible turbulence (LTP), par exemple une pompe péristaltique.

**7.** Procédé selon la revendication 6, dans lequel le système comprend :

une première et une deuxième LTP, dans lequel la première LTP permet l'écoulement de la culture cellulaire dans la première direction et la deuxième LTP permet l'écoulement de la culture cellulaire dans la deuxième direction ; ou
une LTP unique, dans laquelle la LTP unique permet l'écoulement de la culture cellulaire dans la première direction pendant le premier et le troisième laps de temps et permet l'écoulement de la culture cellulaire dans la deuxième direction pendant le deuxième laps de temps.

**8.** Procédé selon la revendication 1, dans lequel le filtrat ne contient pas de cellule mammifère.

**9.** Procédé selon la revendication 1, dans lequel la culture cellulaire contient une protéine recombinante secrétée, par exemple un anticorps ou un fragment de liaison d'antigène correspondant, un facteur de croissance, une cytokine, ou une enzyme, et le filtrat contient la protéine recombinante secrétée.

**10.** Système de filtration à circuit ouvert comprenant (1) un bioréacteur, (2) une unité de filtration à flux tangentiel (TFF) présentant une première et une deuxième entrées, (3) un premier conduit en communication fluidique entre le bioréacteur et la première entrée d'unité TFF, (4) un deuxième conduit en communication fluidique entre le bioréacteur et la deuxième entrée de l'unité TFF, et (5) au moins une pompe disposée dans le système, dans lequel l'actionnement de l'au moins une pompe permet l'écoulement de fluide de manière réversible à travers le système depuis le bioréacteur, à travers le premier conduit, l'unité TFF, le deuxième conduit et de nouveau au réservoir, et éventuellement, comprenant en outre (6) un réservoir de retenue de filtrat et un conduit de filtrat en communication fluidique entre l'unité TFF et le réservoir de retenue de filtrat.

**11.** Système de filtration à circuit ouvert selon la revendication 10, dans lequel le système comprend une ou plusieurs unités TFF supplémentaires disposées dans le premier conduit, le deuxième conduit ou les deux.

**12.** Système de filtration à circuit ouvert selon la revendication 10, dans lequel l'au moins une pompe est : disposée dans le premier conduit ou le deuxième conduit, ou les deux ; ou disposée dans le bioréacteur et proche du premier ou du deuxième conduit de fluide

**13.** Système de filtration à circuit ouvert selon l'une quelconque des revendications 10 à 12, dans lequel l'au moins une pompe est une pompe à faible turbulence (LTP), par exemple une pompe péristaltique.

**14.** Système de filtration à circuit ouvert selon la revendication 13, dans lequel le système comprend :

une première et une deuxième LTP, dans lequel la première LTP est adaptée pour l'écoulement de la culture cellulaire dans une première direction d'écoulement et la deuxième LTP est adaptée pour inverser la première direction d'écoulement et permettre l'écoulement de la culture cellulaire dans la deuxième direction ; ou
une LPT unique adaptée pour permettre l'écoulement de manière réversible de la culture cellulaire dans une première et une deuxième directions d'écoulement.

15. Procédé selon l'une quelconque des revendications 1 à 9, dans lequel le temps de séjour extérieur de la culture cellulaire est compris entre 1 seconde et 20 secondes.

FIG. 1

FIG. 2

FIG. 3

FIG. 4

FIG. 5

FIG. 6

FIG. 7

FIG. 8

FIG. 9

FIG. 10

FIG. 11

FIG. 12

FIG. 13

FIG. 14

FIG. 15

FIG. 16

FIG. 17

FIG. 18

FIG. 19

FIG. 20

FIG. 21

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 20110111486 A
- WO 2014051503 A
- US 4037984 A
- US 5033943 A
- US 5458459 A
- US 20090199904
- WO 06021873 A
- US 20120164066

**Non-patent literature cited in the description**

- **GEBAUER et al.** *Current Opin. Chem. Biol.,* 2009, vol. 13, 245-255